# EUROPEAN PATENT SPECIFICATION

(11) **EP 2 988 714 B1**
(45) Date of publication and mention of the grant of the patent: **17.05.2017**
(21) Application number: 14719506.9
(22) Date of filing: 02.04.2014
(51) Int. Cl.: A61F 13/496, A61F 13/15

(54) **METHODS AND APPARATUSES FOR ASSEMBLING DISPOSABLE DIAPER PANTS**
VERFAHREN UND VORRICHTUNGEN ZUR HERSTELLUNG VON EINWEGWINDELN
PROCÉDÉS ET APPAREILS POUR L'ASSEMBLAGE DE COUCHES-CULOTTES JETABLES

(30) Priority: 26.04.2013 US 201313871017
(43) Date of publication of application: 02.03.2016
(73) Proprietor: The Procter & Gamble Company, Cincinnati, OH 45202 (US)
(72) Inventor: LAVON, Gary, Dean, Cincinnati, Ohio 45202 (US); GOYETTE, Nicholas, Paul, Cincinnati, Ohio 45202 (US); LAM, Joseph, Hung, Bihn Duong 70000 (VN); ZINK, Ronald, Joseph, II, Cincinnati, Ohio 45202 (US)
(74) Representative: Kremer, Véronique Marie Joséphine
(86) International application number: PCT/US2014/032623
(87) International publication number: WO 2014/176007

(56) References cited:
- EP-A1- 2 394 619
- WO-A1-2007/070077

## Description

### FIELD OF THE INVENTION

The present disclosure relates to methods and apparatuses for assembling disposable absorbent articles, and more particularly, relates to methods and apparatuses for assembling disposable diaper pants.

### BACKGROUND OF THE INVENTION

Along an assembly line, various types of articles, such as diapers and other absorbent articles, may be assembled by adding components to and/or otherwise modifying an advancing, continuous web of material. In some processes, advancing webs of material are combined with other advancing webs of material. In other processes, individual components created from advancing webs of material are combined with advancing webs of material, which in turn, are then combined with other advancing webs of material. In some cases, individual components created from advancing web or webs are combined with other individual components created from other advancing web or webs. Webs of material and component parts used to manufacture diapers may include: backsheets, topsheets, leg cuffs, waist bands, absorbent core components, front and/or back ears, front and/or back belts, fastening components, and various types of elastic webs and components such as leg elastics, barrier leg cuff elastics, stretch side panels, and waist elastics.

In some converting processes, chassis spaced apart from each other are advanced in a machine direction and are arranged with a longitudinal centerline parallel with a cross machine direction. A back waist region of the chassis may be joined with a continuous length of elastically extendable waistband web or belt advancing in the machine direction. While connected with the chassis, the waistband web may be maintained in a fully stretched condition along the machine direction, forming a continuous length of absorbent articles. The waistband web may be cut between adjacent chassis to form discrete absorbent articles having a discrete waistband web. The chassis may be folded to bring the front and back waist regions into a face-to-face relationship. First and second end regions of the discrete waistband may be folded to contact the front waist region of the absorbent article and the discrete waistband or belt may be joined with the front waist region of the chassis. The discrete absorbent articles may be advanced to a packaging operation. In some processes, the steps of cutting the waistband web and folding the chassis and the discrete waistband may limit the production rate of the absorbent article assembly line. Therefore, it would be beneficial to provide a process and apparatus for increasing the rate at which these types of absorbent articles may be produced.

### SUMMARY OF THE INVENTION

Aspects of the present disclosure include a method for assembling disposable absorbent articles, each absorbent article comprising a chassis, a first side panel, and a second side panel, each chassis defining a longitudinal axis and a lateral axis, the chassis having a first waist region, a second waist region, and a crotch region positioned longitudinally between the first and second waist regions, the method comprising: advancing a first continuous web in a machine direction to a drum, wherein the drum rotates about a rotation axis, and wherein the drum comprises a first lane of folding devices and a second lane of folding devices in an outer surface of the drum, wherein the first lane of folding devices are offset from the second lane of folding devices in a direction parallel with the rotation axis, wherein each folding device comprises: a chassis support member, a chassis folding member, a first side panel folding member, and a second side panel folding member; positioning the first continuous web on the chassis support member, the first side panel folding member, and the second side panel folding member of the first lane of folding devices; advancing a second continuous web in the machine direction to the drum; positioning the second continuous web on the chassis support member, the first side panel folding member, and the second side panel folding member of the second lane of folding devices; advancing a first chassis in the machine direction to the first lane of folding devices; positioning the first waist region of the first chassis on the chassis support member and the second waist region of the first chassis on the chassis folding member; advancing a second chassis in the machine direction to the second lane of folding devices; positioning the first waist region of the second chassis on the chassis support member and the second waist region of the second chassis on the chassis folding member; connecting the first waist region of the first chassis with the first continuous web; connecting the first waist region of the second chassis with the second continuous web; cutting the first continuous web into discrete lengths of belt material, wherein a portion of the discrete length of belt material supported by the first side panel folding member defines a first side panel, and a portion of the discrete length of belt material supported by the second side panel folding member defines a second side panel; cutting the second continuous web into discrete lengths of belt material, wherein a portion of the discrete length of belt material supported by the first side panel folding member defines a first side panel, and a portion of the discrete length of belt material supported by the second side panel folding member defines a second side panel; pivoting the chassis folding member relative to the chassis support member to position the first and second waist regions of the first chassis into a face-to-face relationship; pivoting the chassis folding member relative to the chassis support member to position the first and second waist regions of the second chassis into a face-to-face relationship; pivoting the first side panel folding member relative to the chassis support member to position the first side panel into contact with the second waist region of the first chassis; pivoting the first side panel folding member relative to the chassis support member to position the first side panel into contact with the second waist region of the second chassis; pivoting the second side panel folding member relative to the chassis support member to position the second side panel into contact with the second waist region of the first chassis; pivoting the second side panel folding member relative to the chassis support member to position the second side panel into contact with the second waist region of the second chassis; connecting the first and second side panels with the second waist region of the first chassis to form a diaper pant; and connecting the first and second side panels with the second waist region of the second chassis to form a diaper pant.

Aspects of the present disclosure include a method for assembling disposable diaper pants, each diaper pant comprising a chassis, a first side panel, and a second side panel, each chassis defining a longitudinal axis and a lateral axis, the chassis having a first waist region, a second waist region, and a crotch region positioned longitudinally between the first and second waist regions, the method comprising: rotating a drum about an axis of rotation, the drum comprising a first lane of folding devices on an outer surface of the drum and a second lane of folding devices on the outer surface of the drum, wherein each folding device comprises: a chassis support member, a chassis folding member, a first side panel folding member, and a second side panel folding member; advancing first and second side panels in a machine direction to the first lane of folding devices, wherein the first side panel is positioned on the first side panel folding member and the second side panel is positioned on the second side panel folding member; advancing a first chassis in the machine direction to the first lane of folding devices, wherein the first waist region of the first chassis is positioned on the chassis support member and the second waist region of the first chassis is positioned on the chassis folding member; connecting first end regions of the first and second side panels with the first waist region of the first chassis; advancing third and fourth side panels in the machine direction to the second lane of folding devices, wherein the third side panel is positioned on the first side panel folding member and the fourth side panel is positioned on the second side panel folding member; advancing a second chassis in the machine direction to the second lane of folding devices, wherein the first waist region of the second chassis is positioned on the chassis support member and the second waist region of the second chassis is positioned on the chassis folding member; connecting first end regions of the third and fourth side panels with the first waist region of the second chassis; pivoting the chassis folding member of the first lane of folding devices relative to the chassis support member to position the first and second waist regions of the first chassis into a face-to-face relationship; pivoting the chassis folding member of the second lane of folding devices relative to the chassis support member to position the first and second waist regions of the second chassis into a face-to-face relationship; pivoting the first and second side panel folding members of the first lane of folding devices relative to the chassis support member to position the first and second side panels into contact with the second waist region of the first chassis; pivoting the first and second side panel folding members of the second lane of folding devices relative to the chassis support member to position the third and fourth side panels into contact with the second waist region of the second chassis; connecting the first and second side panels with the second waist region of the first chassis to form a first diaper pant; and connecting the second and third side panels with the second waist region of the second chassis to form a second diaper pant.

Aspects of the present disclosure include an apparatus for assembling diaper pants comprising a drum rotatable about an axis of rotation. The drum has an outer surface comprising a first lane of folding devices. Each folding device comprises a chassis support member; a chassis folding member pivotally connected with the chassis support member; a first side panel folding member pivotally connected with the chassis support member; and a second side panel folding member pivotally connected with the chassis support member. The drum comprises a second lane of folding devices, each folding device comprising: a chassis support member; a chassis folding member pivotally connected with the chassis support member; a first side panel folding member pivotally connected with the chassis support member; and a second side panel folding member pivotally connected with the chassis support member, wherein the first lane of folding devices is offset from the second lane of folding devices in a direction parallel to the axis of rotation.

### BRIEF DESCRIPTION OF THE DRAWINGS

Fig. 1 is a schematic, plan view of an unfolded diaper pant.
Fig. 2 is a schematic, plan view of a diaper pant having a bi-folded chassis.
Fig. 3 is a schematic, plan view of a folded diaper pant having folded side panels permanently fastened with the chassis.
Fig. 4 is a schematic, plan view of a folded diaper pant having side panels refastenably connected with a chassis.
Fig. 5 is a schematic, plan view of an unfolded diaper pant having discrete side panels.
Fig. 6A is a schematic, plan view of an unfolded diaper pant.
Fig. 6B is a schematic, plan view of a folded diaper pant.
Fig. 7 is a schematic, plan view of an unfolded tape-style diaper.
Fig. 8 is a schematic, elevation view of a converting apparatus for assembling and folding diaper pants.
Fig. 9 is a schematic, plan view of two continuous lengths of unfolded absorbent articles taken along line 9-9 of Fig. 8.
Fig. 10 is a schematic, plan view of two continuous lengths of absorbent articles having bi-folded chassis taken along 10-10 of Fig. 8.
Fig. 11 is a schematic, plan view of two lanes of folded, discrete absorbent articles taken along line 11-11 of Fig. 8.
Fig. 12 is a perspective view of a folded, discrete diaper pant.
Fig. 13 is a schematic, plan view of a continuous length of chassis.
Fig. 14 is a schematic, plan view of a chassis in a first orientation.
Fig. 15 is a schematic, plan view of a chassis in a second orientation.
Fig. 16 is a perspective view of a folding drum having first and second lanes of folding devices.
Fig. 17 is a plan view of a folding drum having first and second lanes of folding devices.
Fig. 18 is a plan view of a folding drum having one lane of folding devices.
Fig. 19 is a perspective view of a folding device having a chassis support member, chassis folding member, and first and second side panel folding members.
Fig. 20 is a perspective view of the folding device of Fig. 19.
Fig. 21 is a perspective view of the folding device of Fig. 19.
Fig. 22 is a perspective view of the folding device of Fig. 19.
Fig. 23 is a perspective view of the folding device of Fig. 19.
Fig. 24 is a perspective view of the folding device of Fig. 19.
Fig. 25 is a perspective view of the folding device of Fig. 19.
Fig. 26 is a perspective view of the folding device of Fig. 19.
Fig. 27 is a schematic, elevation view of a converting apparatus.
Fig. 28 is a linear, plan view of a folding drum having two lanes of folding devices.
Fig. 29 is a linear, plan view of a folding drum having two lanes of folding devices.
Fig. 30 is a linear, plan view of a folding drum having two lanes of folding devices.
Fig. 31 is a linear, plan view of a folding drum having two lanes of folding devices.
Fig. 32 is a linear, plan view of a folding drum having two lanes of folding devices.
Fig. 33 is a schematic, elevation view of a converting apparatus.
Fig. 34 is a schematic, elevation view of a converting apparatus.

### DETAILED DESCRIPTION OF THE INVENTION

The following definitions may be useful in understanding the present disclosure:

"Absorbent article" is used herein to refer to consumer products whose primary function is to absorb and retain soils and wastes. "Diaper" is used herein to refer to an absorbent article generally worn by infants and incontinent persons about the lower torso. The term "disposable" is used herein to describe absorbent articles which generally are not intended to be laundered or otherwise restored or reused as an absorbent article (for example, they are intended to be discarded after a single use and may also be configured to be recycled, composted or otherwise disposed of in an environmentally compatible manner).

"Longitudinal" means a direction running substantially perpendicular from a waist edge to a longitudinally opposing waist edge of an absorbent article when the article is in a flat out, uncontracted state, or from a waist edge to the bottom of the crotch, i.e. the fold line, in a bi-folded article. Directions within 45 degrees of the longitudinal direction are considered to be "longitudinal." "Lateral" refers to a direction running from a longitudinally extending side edge to a laterally opposing longitudinally extending side edge of an article and generally at a right angle to the longitudinal direction. Directions within 45 degrees of the lateral direction are considered to be "lateral."

"Substrate" is used herein to describe a material which is primarily two-dimensional (i.e. in an XY plane) and whose thickness (in a Z direction) is relatively small (i.e. 1/10 or less) in comparison to the substrate's length (in an X direction) and width (in a Y direction). Nonlimiting examples of substrates include a web, layer or layers or fibrous materials, nonwovens, films and foils such as polymeric films or metallic foils. These materials may be used alone or may comprise two or more layers joined together. As such, a web is a substrate.

"Nonwoven" refers herein to a material made from continuous (long) filaments (fibers) and/or discontinuous (short) filaments (fibers) by processes such as spunbonding, meltblowing, carding, and the like. Nonwovens do not have a woven or knitted filament pattern.

"Machine direction" (MD) is used herein to refer to the direction of material flow through a process. In addition, relative placement and movement of material can be described as flowing in the machine direction through a process from upstream in the process to downstream in the process.

"Cross machine direction" (CD) is used herein to refer to a direction that is not parallel with, and usually perpendicular to, the machine direction.

"Pant" (sometimes referred to in commerce as "training pant", "pre-closed diaper", "diaper pant", "pant diaper", and "pull-on diaper") refers herein to disposable absorbent articles having a continuous perimeter waist opening and continuous perimeter leg openings designed for infant or adult wearers. A pant can be configured with a continuous or closed waist opening and at least one continuous, closed, leg opening prior to the article being applied to the wearer. A pant can be preformed by various techniques including, but not limited to, joining together portions of the article using any refastenable and/or permanent closure member (for example, seams, heat bonds, pressure welds, adhesives, cohesive bonds, mechanical fasteners, etc.). A pant can be preformed anywhere along the circumference of the article in the waist region (for example, side fastened or seamed, front waist fastened or seamed, rear waist fastened or seamed).

"Pre-fastened" refers herein to pant diapers manufactured and provided to consumers in a configuration wherein the front waist region and the back waist region are fastened or connected with each other as packaged, prior to being applied to the wearer. As such pant diapers may have a continuous perimeter waist opening and continuous perimeter leg openings designed for infant or adult wearers. As discussed in more detail below, a diaper pant can be preformed by various techniques including, but not limited to, joining together portions of the diaper using refastenable and/or permanent closure members (for example, seams, heat bonds, pressure welds, adhesives, cohesive bonds, mechanical fasteners, etc.). In addition, pant diapers can be pre-fastened anywhere along the circumference of the waist region (for example, side fastened or connected, front waist fastened or connected, rear waist fastened or connected).

"Elastic," "elastomer" or "elastomeric" refers to any material that upon application of a force to the material's relaxed, initial length can stretch or elongate to an elongated length more than 10% greater than the material's initial length and will substantially recover back to about the material's initial length upon release of the applied force. The term "inelastic" refers herein to any material that does not fall within the definition of "elastic".

Aspects of the present disclosure involve methods and apparatuses for manufacturing a variety of absorbent articles, and more particularly, methods and apparatuses for assembling diaper pants. Exemplary absorbent articles include a chassis, a waistband, a first side panel, and a second side panel. Each chassis may comprise a topsheet, a backsheet, and an absorbent core disposed between the topsheet and the backsheet. The chassis defines a longitudinal axis and a lateral axis. Each chassis may define a first waist region, a second waist region, and a crotch region disposed between the first and second waist regions. The first and second side panels may connect the first and second waist regions to form a waist opening and a pair of leg openings prior to packaging or prior to being donned on the wearer. The chassis comprises a garment-facing surface and a wearer-facing surface.

An apparatus for assembling absorbent articles may comprise a folding drum rotatable about an axis of rotation and having an outer surface. The outer surface of the folding drum may comprise first and second lanes of folding devices. Each folding device comprises a chassis support member, a chassis folding member pivotally connected with the chassis support member, a first side panel folding member pivotally connected with the chassis support member, and a second side panel folding member pivotally connected with the chassis support member. The first lane of folding devices may be offset from the second lane of folding devices in a direction parallel to the axis of rotation. In some exemplary configurations, the chassis support members, chassis folding members, first side panel folding members, and second side panel folding members of the first and second lane of folding devices each comprise a zone of vacuum apertures.

In an exemplary folding drum having first and second lanes of folding devices, a first continuous web may advance in a machine direction to the first lane of folding devices and a second continuous web may advance in the machine direction to the second lane of folding devices. The first and second continuous webs are positioned on the chassis support member, the first side panel folding member, and the second side panel folding member of the folding devices in the first and second lanes, respectively. In addition, a first chassis is advanced in the machine direction to the first lane of folding devices and a second chassis is advanced in the machine direction to the second lane of folding devices. The first waist regions of the first and second chassis are positioned on the chassis support member of the folding devices of the first and second lanes, respectively. The second waist regions of the first and second chassis are positioned on the chassis folding member of the folding devices of the first and second lanes, respectively. The first waist region of the first chassis is connected with the first continuous web. The first waist region of the second chassis is connected with the second continuous web. The first and second chassis may be disposed on the folding drum prior to, or subsequent to, the first and second continuous web advancing onto the folding drum. The first and second continuous webs may be connected with the chassis prior to, or subsequent to, the first and second continuous webs advancing onto the folding drum.

Next, the first continuous web is cut between adjacent folding devices into first discrete waist belts. A portion of the first discrete waist belt supported by the first side panel folding member defines a first side panel and a portion of the first discrete waist belt supported by the second side panel folding member defines a second side panel. The second continuous web is then cut between adjacent folding devices into second discrete waist belts. A portion of the second discrete waist belt supported by the first side panel folding member defines a third side panel and a portion of the second discrete waist belt supported by the second side panel folding members defines a fourth side panel.

In some exemplary configurations of a pant absorbent article, the first chassis is then bi-folded by pivoting the chassis folding member of the first lane of folding devices relative to the chassis support member to position the first and second waist regions of the first chassis into a face-to-face relationship. The second chassis is bi-folded by pivoting the chassis folding member of the second lane of folding devices relative to the chassis support member to position the first and second waist regions of the second chassis into a face-to-face relationship.

The first and second side panels are then folded by pivoting the first and second side panel folding members of the first lane of folding devices relative to the chassis support member to position the first and second side panels into contact with the second waist region of the first chassis. Next, the third and fourth side panels are folded by pivoting the first and second side panel folding members of the second lane of folding devices relative to the chassis support member to position the third and fourth side panels into contact with the second waist region of the second chassis. The first and second side panels are connected with the second waist region of the first chassis to form a first diaper pant. The third and fourth side panels are connected with the second waist region of the second chassis to form a second diaper pant. The first and second diaper pants are discharged from the drum and advanced to a packaging operation.

In some exemplary configurations, the first, second, third, and fourth side panels, may advance to the folding drum as individual components. For example, the first continuous web may be cut into first and second side panels prior to the first and second side panels advancing onto the folding drum. Likewise, the second continuous web may be cut into third and fourth side panels prior to the third and fourth side panels advancing onto the drum folding.

The folding device may bi-fold the chassis prior to, or subsequent to, folding the side panels. If it is desired to attach the side panels to the garment-facing surface of the chassis, the chassis is first bi-folded on the drum and then the side panels are folded, either sequentially or generally concurrently. Comparatively, if it is desired to attach the side panels to the wearing-facing surface of the chassis, for example in a taped diaper absorbent article context, the side panels are first folded on the rotating drum, either sequentially or generally concurrently, and then the chassis is bi-folded on the drum.

Furthermore, in some exemplary configurations, the bi-folding process and the side panel folding process may at least partially overlap. For example, if the chassis is bi-folded before the side panels are folded, the bi-folding process does not have to completely finish before the side panel folding process begins. Similarly, if the side panels are folded before the chassis is bi-folded, the side panel folding process does not have to completely finish before the chassis bi-folding begins. By allowing at least some of the folding process to overlap, the amount of time needed for one absorbent article to be folded along multiple fold lines may be decreased.

To help provide additional context to the subsequent discussion, the following provides a general description of absorbent articles in the form of taped diapers and diaper pants that may be manufactured in accordance with the methods and apparatuses disclosed herein. While the following disclosure references primarily disposable diaper pants, and it to be appreciated that the methods and apparatuses disclosed herein may be used to assemble various forms of disposable absorbent articles, including various forms of taped diapers, adult incontinence articles, and sanitary napkins.

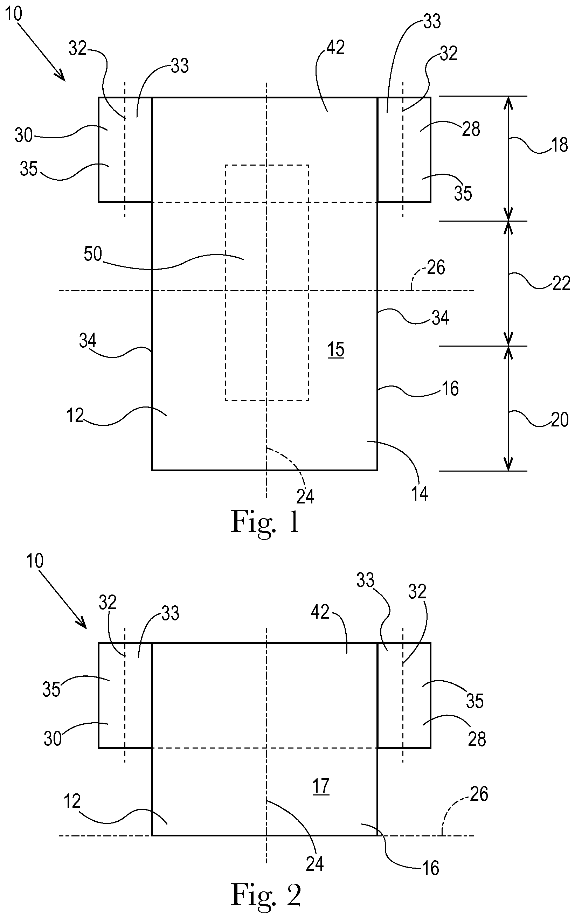

As shown in Figs. 1 and 2, an exemplary diaper pant 10 may comprise a chassis 12 comprising a topsheet 14 forming at least a portion of a wearer-facing surface 15, a backsheet 16 forming at least a portion of a garment-facing surface 17, and an absorbent core 50 disposed between the topsheet 14 and the backsheet 16. The chassis 12 may comprise a first waist region 18 longitudinally opposed to a second waist region 20 and a crotch region 22 positioned between the first and second waist regions 18 and 20. Each chassis 12 may have a longitudinal axis 24 and a lateral axis 26. In some exemplary configurations, a first side panel 28 and a second side panel 30 may extend laterally outward from the first waist region 18 or, in other exemplary configurations, the first side panel 28 and the second side panel 30 may extend laterally outward from the second waist region 20. In addition the web comprising the first and second side panels may extend longitudinally beyond the end edge of the chassis to form a continuous article waist edge or waistband. As shown in Fig. 2, the chassis 12 may be bi-folded about the lateral axis 26 such that the first waist region 18 is in a face-to-face relationship with the second waist region 20.

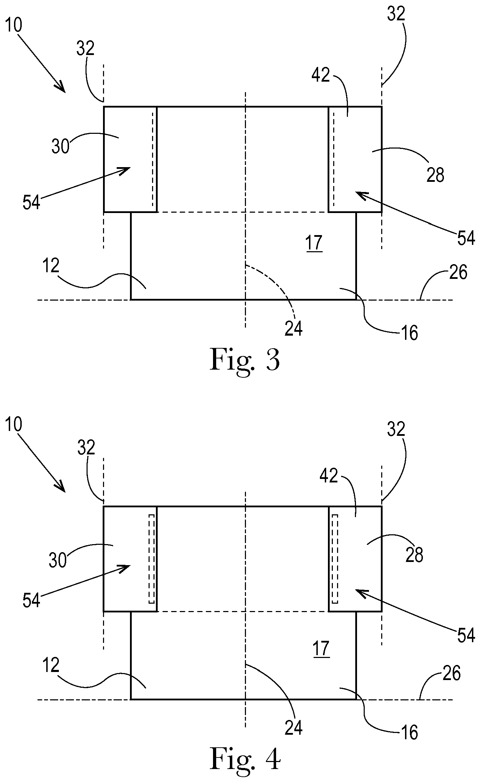

With reference to Figs. 1-3, the first side panel 28 and the second side panel 30 may each define a fold line 32. Second end regions 35 of the first and second side panels 28 and 30 may be folded laterally inward about the fold lines 32 over a first end region 33 of the first and second side panels 28 and 30. In some exemplary configurations, the fold lines 32 are not structurally incorporated into the first side panel 28 and the second side panel 30. Instead, the first side panel 28 and the second side panel 30 may be generally homogenous in structure and not prone to, or otherwise configured to, fold along any particular line. As shown in Figs. 3 and 4, the first and second side panels 28 and 30 may be folded laterally inward about the fold lines 32. The first and second side panels 28 and 30 may be connected with the second waist region 20 of the chassis 12 at attachment regions 54. The first and second side panels 28 and 30 may be permanently connected with the second waist region 20 of the chassis 12 as shown in Fig. 3. In other exemplary configurations, the first and second side panels 28 and 30 may be refastenably connected with the second waist region 20 of the chassis 12 such as shown in Fig. 4. The first and second side panels may be connected to the inner, wearer facing surface or the outer, garment facing surface of the second waist region of the chassis.

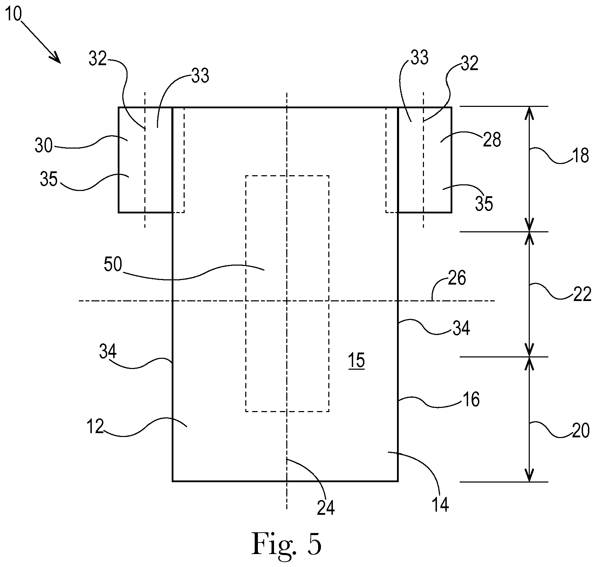

In various exemplary configurations, the first and second side panels 28 and 30 may be formed of a single component, shown as a discrete waist belt 42 in Figs. 1-3. The discrete waist belt 42 forming the first and second side panels 28 and 30 may be attached to the garment facing surface 17 of the first waist region 18 as shown in Figs. 1-3. In some exemplary configurations, the discrete waist belt 42 may be attached to the wearer facing surface 15 of the first waist region 18, for example. When joined to the interior surface 15 of the chassis the central portion of the discrete waist belt may remain partially free of attachment to the interior surface to provide a pocket at the waist for trapping of bodily waste. In other exemplary configurations, as shown in Fig. 5, first and second side panels 28 and 30 may be in the form of discrete components. The first and second side panels 28 and 30 may be attached adjacent the laterally opposing side edges 34 of the chassis 12 in the first waist region 18 as shown in Fig. 5. The first and second side panels 28 and 30 may be attached to the wearer facing surface 15 of the chassis 12, or may be attached to the garment facing surface 17 of the chassis 12.

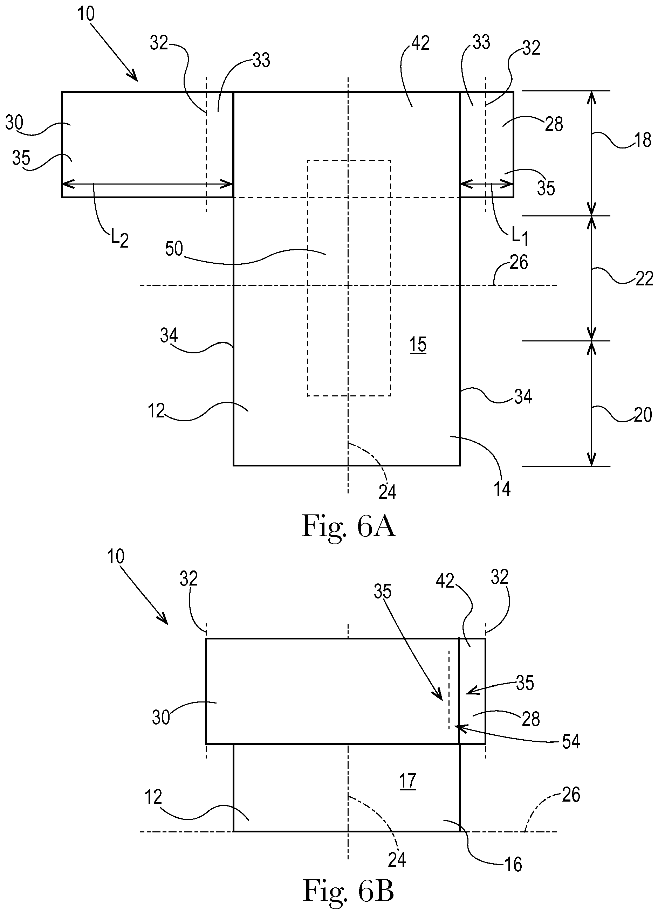

In some exemplary configurations, the first and second side panels 28 and 30 may have different dimensions. For example, as shown in Fig. 6A, a first side panel 28 may have a length, L₁, and a second side panel 30 may have a length, L₂. The length, L₂, of the second side panel 30 may be greater than the length, L₁, of the first side panel 28. In such a configuration, as shown in Fig. 6B, the first and second side panels 28 and 30 may be folded about fold lines 32 such that the second side panel 30 extends laterally along the second waist region 20 of the chassis 12. The second end region 35 of the second side panel 30 may be joined with the second end region 35 of the first side panel 28. In such an exemplary configuration, the waist belt may wrap the entirety of the waist circumference when donned. The chassis or the waist belt may be connected to each other in multiple locations in the area of overlap of the side panel 30 and the chassis 12.

In some exemplary configurations, the first and second side panels 28 and 30 may comprise elastic materials that have been "prestrained" or "mechanically prestrained" (i.e., subjected to some degree of localized pattern mechanical stretching to permanently elongate the material). The materials may be prestrained using suitable deep embossing techniques. In other exemplary configurations, the materials may be prestrained by directing the material through an incremental mechanical stretching system as described in U.S. Patent No. 5,330,458. The materials may then be allowed to return to their substantially untensioned condition, thus forming a zero strain stretch material that is extensible, at least up to the point of initial stretching. Examples of zero strain materials are disclosed in U.S. Patent Nos. 2,075,189, 3,025,199, 4,107,364, 4,209,563, 4,834,741, and 5,151,092.

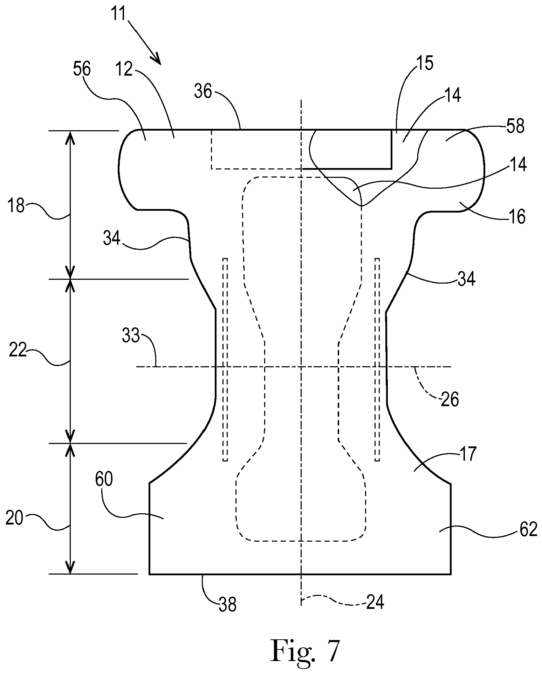

Fig. 7 shows an exemplary taped style diaper 11. The diaper 11 includes a chassis 12 having a first ear 56, a second ear 58, a third ear 60, and a fourth ear 62. To provide a frame of reference for the present discussion, the chassis is shown with a longitudinal axis 26 and a lateral axis 28. The chassis 12 is shown as having a first waist region 18, a second waist region 20, and a crotch region 22 disposed intermediate the first and second waist regions 18 and 20. The periphery of the diaper 11 is defined by a pair of longitudinally extending side edges 34; a first end edge 36 extending laterally adjacent the first waist region 18; and a second end edge 35 extending laterally adjacent the second waist region 20. As shown in FIG. 7, the chassis 12 includes an inner, wearer-facing surface 15, and an outer, garment-facing surface 17. A portion of the chassis 12 is cut-away in FIG. 1 to more clearly show the construction of and various features that may be included in the diaper. As shown in FIG. 7, the chassis 12 of the diaper 11 may include a topsheet 14 and a backsheet 16. An absorbent core 65 may be disposed between a portion of the topsheet 14 and the backsheet 16. Any one or more of the regions may be stretchable and may include an elastomeric material or laminate material. As such, the diaper 11 may be configured to adapt to a specific wearer's anatomy upon application and to maintain coordination with the wearer's anatomy during wear.

With continuing reference to Fig. 7, although the first and second ears 56, 58 as well as the third and fourth ears 60, 62 are illustrated as being integrally formed with the chassis 12, it is to be appreciated that other embodiments may include ears that are discrete elements connected with the chassis 12. In some embodiments, the ears are configured to be stretchable. The ears may also include one or more elastomeric elements as well as one or more fastener elements adapted to releasably connect with each other and/or other fastener elements on the chassis. In certain embodiments the diaper may comprise only first and second ears disposed in one of the waist regions. A more detailed discussion of stretchable ears can be found in U.S. Patent Nos. 4,857,067; 5,151,092; 5,674,216; 6,677,258; 4,381,781; 5,580,411; and 6,004,306. The ears may also include various geometries and arrangements of stretch zones or elements, such as discussed in U.S. Pat. Publication Nos. US2005/0215972A1 and US2005/0215973A1.

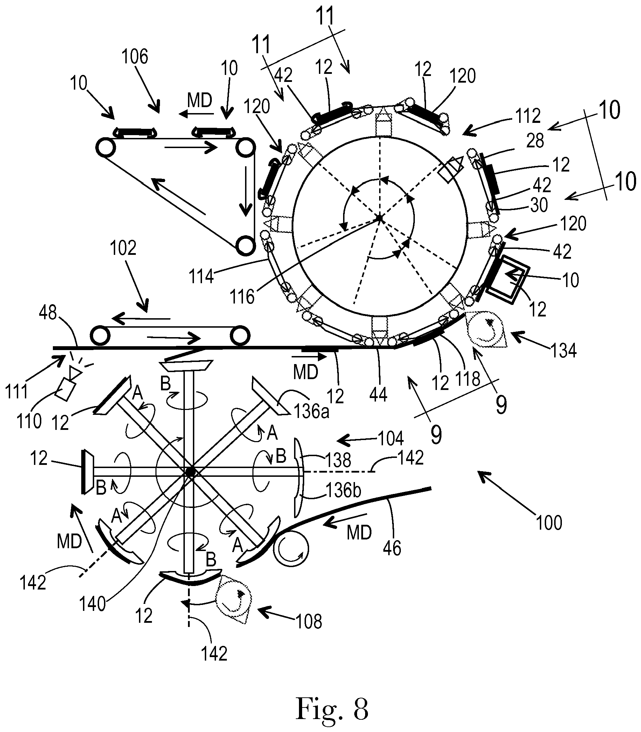

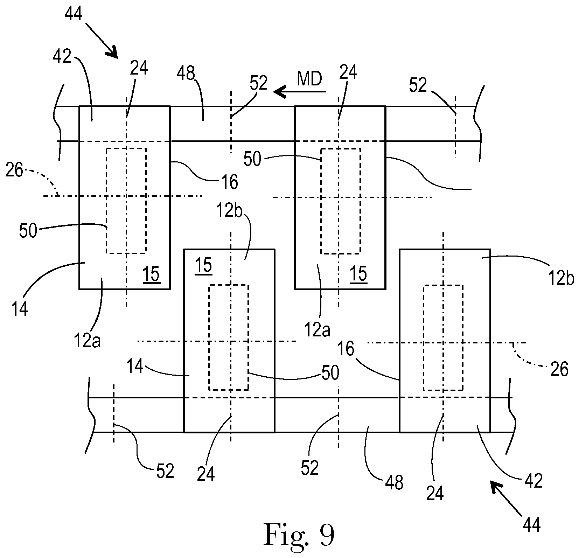

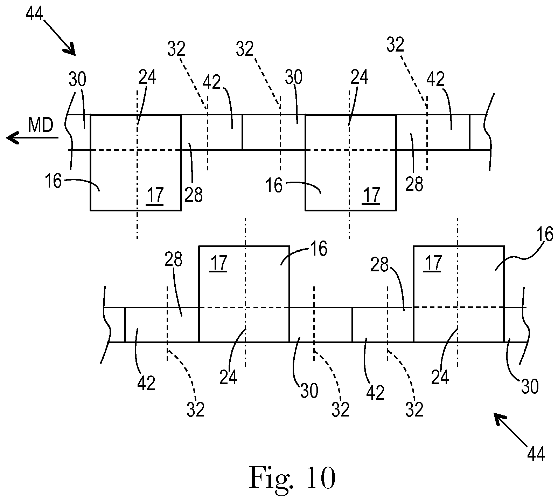

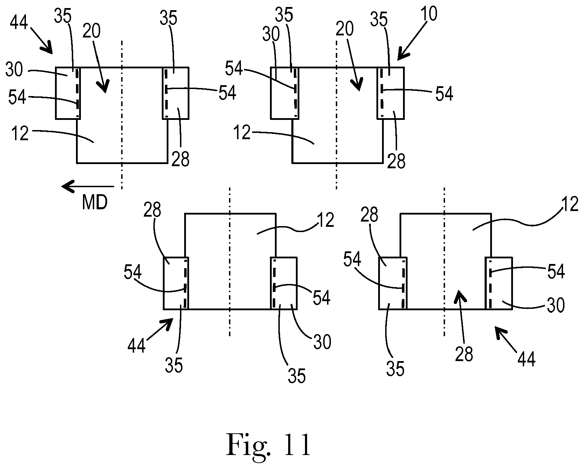

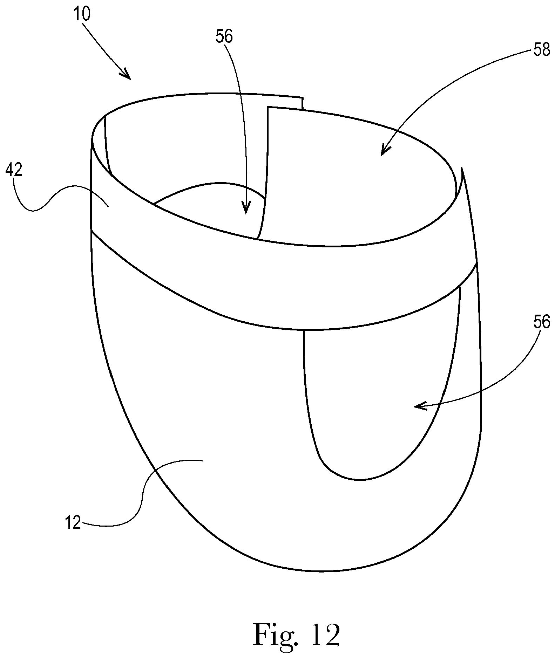

Fig. 8 shows an exemplary converting apparatus 100 that is adapted to assemble diaper pants. In operation, a first continuous web, shown as a continuous waist belt web 48 advances in a machine direction MD to a folding drum 112. A chassis 12 may also advance in the machine direction MD and may be combined with the continuous waist belt web 48 to form a continuous length of unfolded absorbent articles 44. As shown in Figs. 1 and 9, the first end regions 33 of the first and second side panels 28 and 30 may be connected with the garment-facing surface 17 of the first waist region 18 of the chassis 12. In other exemplary configurations, the first and second side panels 28 and 30 may be connected with the wearer-facing surface 15 of the first waist region 18. Next, the continuous waist belt web 48 may be cut along cut lines 52 shown in Fig. 5 into discrete waist belts 42. Then, referring to Figs. 2 and 10, the chassis 12 may be folded about the lateral axis 26 to position the first and second waist regions 18 and 20 of the chassis 12 in a face-to-face relationship. With reference to Figs. 3 and 11, the second end regions 35 of the first and second side panels 28 and 30 are folded laterally inward toward the longitudinal axis 24 about the fold line 32 and are connected with the second waist region 20 at an attachment region 54. As a result, a waist opening 58 and two leg openings 56 are formed in the diaper pant 10 as shown in Fig. 12. The diaper pant 10 is then discharged from the folding drum 112 and advanced in the machine direction MD to a packaging operation.

As shown in Fig. 8, in some exemplary configurations, a continuous waist belt web 48 may advance in a machine direction MD on a first carrier apparatus 102 and may be intermittently combined with chassis 12 advancing in the machine direction MD on a second carrier apparatus 104 to form a continuous length of unfolded absorbent articles 44. The second carrier apparatus 104 may rotate about a first axis of rotation 140. The second carrier apparatus 104 comprises a plurality of first carrier members 136a separated by a plurality of second carrier apparatus 136b, wherein each carrier member 136a and 136b comprises an outer surface 138. The first carrier members 136a rotate in a first direction A about a second axis of rotation 142 and the second carrier members 136b rotate in a second direction B about the second axis of rotation 142. The first direction A is opposite the second direction B and the second axis of rotation 142 is orthogonal to the first axis of rotation 140. As shown in Fig. 8, the second axis of rotation 142 may be offset from the center of the carrier members 136a and 136b.

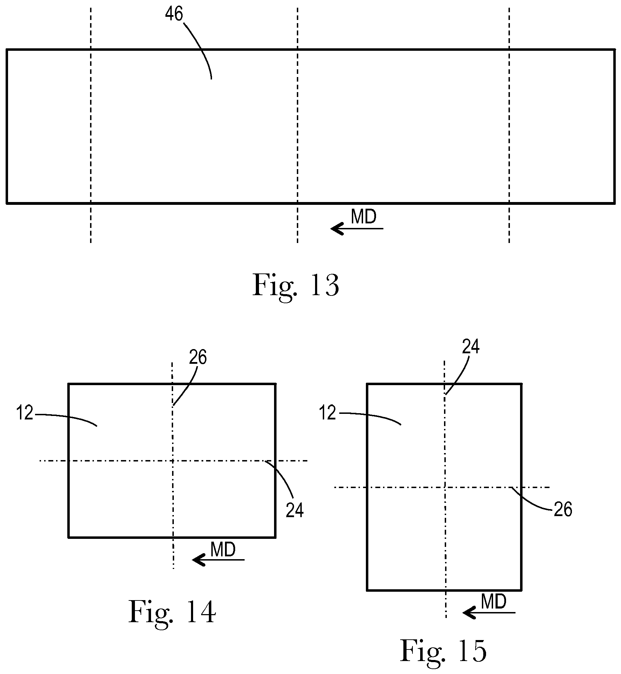

With reference to Figs. 8, 13 and 14, a continuous length of chassis 46 may advance in the machine direction MD on the outer surface of the carrier members of the second carrier apparatus 104 and may be cut by a cutting member 108 into chassis 12. The second carrier apparatus 104 may advance the chassis 12 in the machine direction MD in a first orientation shown in Fig. 14 where the longitudinal axis 24 is parallel with the machine direction MD. The second carrier apparatus 104 may also reorient the chassis 12 to a second orientation shown in Fig. 15 where the lateral axis 26 is parallel with the machine direction MD. More particularly, a first chassis 12a advancing on the first carrier members 136a may rotate in the first direction B about the second axis of rotation 142 to the orientation shown in Fig. 9 and a second chassis 12b advancing on the second carrier members 136b may rotate in the second direction about the second axis of rotation 142 to the orientation shown in Fig. 9. As a result of the second axis of rotation 142 being offset from the center of the carrier members 136a and 136b, the first chassis 12a are offset from the second chassis 12b. That is, the lateral axis 24 of the first chassis 12a is offset from the lateral axis 24 of the second chassis 12b. In addition, the second carrier apparatus 104 may change the speed at which the chassis 12 advances in the machine direction MD. The chassis 12 may be transferred from the second carrier apparatus 104 to the continuous waist belt web 48. The continuous waist belt web 48 may be joined with either the wearer-facing surface 15 or the garment-facing surface 17 of the chassis 12. The continuous waist belt web 48 may be joined with the chassis 12 in a variety of ways. For example, as shown in Fig. 4, adhesive 111 may be applied to the continuous waist belt web 48 using an adhesive applicator 110. The continuous length of unfolded absorbent articles 44 may advance in the machine direction MD to a folding drum 112.

It is to be appreciated that the first and second carrier apparatuses 102 and 104 may be configured in various ways. The first and/or second carrier apparatuses 102 and 104 may be in the form of conveyors, drums, and the like. The first and second carrier apparatuses 102 and 104 may be configured to apply vacuum to the continuous waist belt web 48 and the chassis 12 to hold the continuous waist belt web 48 and the chassis 12 on the first and second carrier apparatuses 102 and 104, respectively. An exemplary carrier apparatus that may be used as the second carrier apparatus is described in U.S. Patent No. 7,587,966.

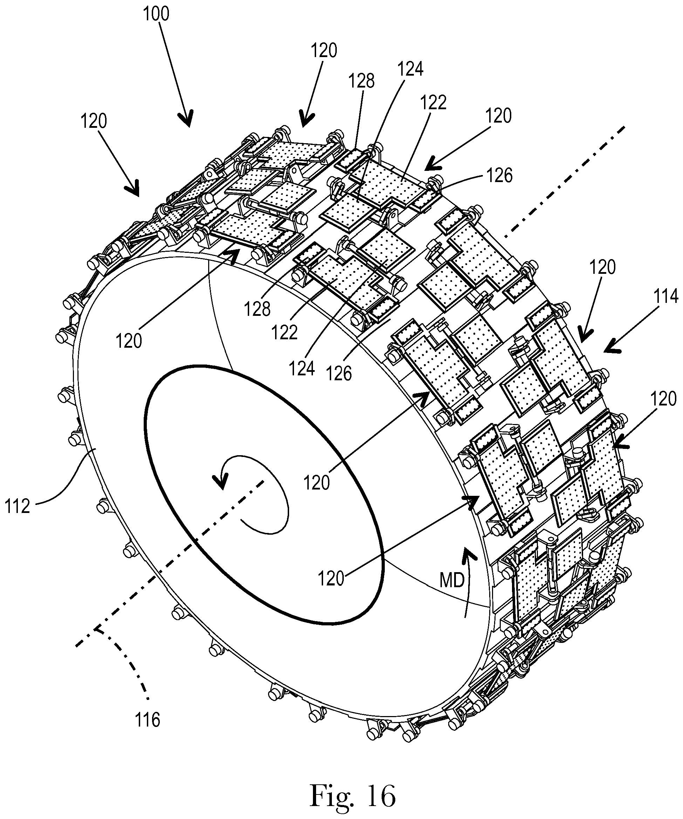

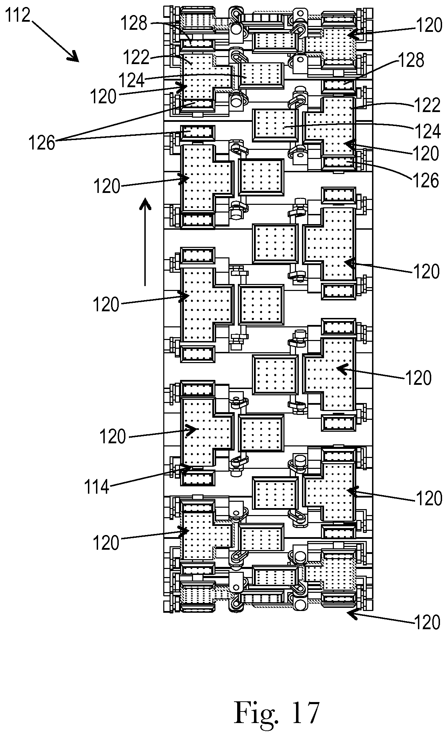

As shown in Fig. 8, from the first carrier apparatus 102, the continuous length of unfolded absorbent articles 44 advances to a folding drum 112. The folding drum 112 is rotatable about an axis of rotation 116 and has an outer surface 114. A plurality of folding devices 120 are positioned on the outer surface 114 of the folding drum 112. The folding drum 112 may comprise first and second lanes 118a and 118b of folding devices 120 such as shown in Figs. 16 and 17. Each folding device 120 may comprise a chassis support member 122 and a chassis folding member 124 pivotally connected with the chassis support member 122. The folding devices 120 may each comprise first and second side panel folding members 126 and 128 that are pivotally connected with the chassis support member 122. The chassis support member 122, chassis folding member 124, and the first and second side panel folding members 126 and 128 may be configured in various ways. For example, the chassis support member 122, chassis folding member 124, and the first and second side panel folding members 126 and 128 may be in the form of plates, frames, and may each comprise one or more arms. The chassis folding member 124 and first and second side panel folding members 126 and 128 may each be configured to pivot relative to the chassis support member 122 using various drive mechanisms. For example, suitable drive mechanisms include mechanical linkages, hydraulic members, cam assemblies, pneumatic members, gearboxes, clutch mechanisms, servo motors, adjacent rollers, belts, and the like.

Each lane 118 of folding devices 120 may be configured to assemble and fold absorbent articles. Thus, while it is shown in Fig. 8 that a first continuous web advances to the folding drum 112, it is to be appreciated that a second continuous web in the form of a second waist belt web may also advance to the folding drum. For example, in an exemplary configuration where a folding drum comprises first and second lanes of folding devices, the first continuous web may advance to the first lane of folding devices and the second continuous web may advance to the second lane of folding devices. Moreover, in such an exemplary configuration, separate chassis may advance to the first and second lanes of folding devices. For example, the chassis 12 shown in Fig. 8 may be in the form of first chassis and may advance to the first lane of folding devices. Second chassis may advance to the second lane of folding devices. As such, a single folding drum 112 may be used to concurrently fold multiple absorbent articles, thereby increasing the production rate of the folding drum 112. It is to be appreciated that the method of operation of the folding devices 120 in the first and second lanes 118a and 118b may be the same or alternatively may be different allowing production of two distinct article forms.

Each of the chassis support member 122, chassis folding member 124, and the first and second side panel folding members 126 and 128 may comprise a zone 132 of vacuum apertures 130 used to hold the continuous length of unfolded absorbent articles 44 on the folding devices 120. Each zone 132 of vacuum apertures 130 may be independently controlled.

During rotation of the folding drum 112, an absorbent article in each lane 118 of folding devices 120 may pass through a variety of stages. As shown in Fig. 8, the diaper pant 10 may rotate through stages A-F, for example. However, it is to be appreciated that some exemplary configurations, the folding drum 112 may rotate a diaper pant 10 through a different number of stages. During some of the stages, various folding devices 120 on the folding drum 112 may sequentially or concurrently actuate to perform the required folds. In some exemplary configurations, some stages may at least partially overlap each other. It is to be appreciated that various stages may occur in different orders from what is described below.

While the following disclosure references a single absorbent article rotating on a single lane of a folding drum, it is to be appreciated that each lane of folding devices may rotate an absorbent article through the same stages. As discussed in more detail below, the folding devices in the second lane may concurrently or sequentially rotate a diaper pant through the same stages as a folding device in the first lane. In some exemplary configurations, the stages of rotation in a first lane of folding devices may partially overlap stages of rotation in a second lane of folding devices.

With continuing reference to Fig. 8, stage A is a portion of the rotation of the folding drum 112 where the continuous length of unfolded absorbent articles 44 is transferred onto the outer surface 114 of the folding drum 112. Stage B is a portion of the rotation of the folding drum 112 where the continuous waist belt web 48 is cut into discrete waist belts 42 while advancing on the folding drum 112. Stage C is a portion of the rotation of the folding drum 112 where the chassis 12 is bi-folded. It is to be appreciated that stages B and C may occur sequentially, or may occur concurrently. Stage D is a portion of the rotation of the folding drum 112 where the first and second side panels 28 and 30 of the discrete waist belts 42 are folded. In some exemplary configurations, stage D may occur prior to stage C. That is, the first and second side panels 28 and 30 may be folded prior to bi-folding of the chassis 12. Stage E is a portion of the rotation where the first and second side panels 28 and 30 are bonded or otherwise attached to the second waist region 120 of the chassis 12. Stage F a portion of the rotation where the diaper pant 10 is discharged from the folding drum 112. The diaper pant 10 may be discharged onto a third carrier apparatus 106, for example.

Referring to Figs. 8, 9, and 16, in stage A, a continuous length of unfolded absorbent articles 44 advances in the machine direction MD onto outer surface 114 of the folding drum 112. In particular, the continuous waist belt web 48 may advance onto a folding device 120 such that the continuous waist belt web 48 is positioned on first side panel folding member 126, the chassis support member 122, and the second side panel folding member 128. The chassis 12 may be positioned such that the first waist region 18 is positioned on the chassis support member 122 and the second waist region 20 is positioned on the chassis folding member 124.

Referring to Fig. 8, in stage B, as the continuous length of unfolded absorbent articles 44 continues advancing in the machine direction MD on the folding drum 112, a cutting member 134 may cut the continuous waist belt web 48 into discrete waist belts 42, thereby forming individual diaper pants 10. As shown in Fig. 8, the cutting member 134 may be in the form of a rotary cutting knife. A portion of the discrete waist belt 42 supported by the first side panel folding member 126 defines a first side panel 28 of the diaper pant 10 and a portion of the discrete waist belt 42 supported by the second side panel folding member 128 defines a second side panel 30 of the diaper pant 10.

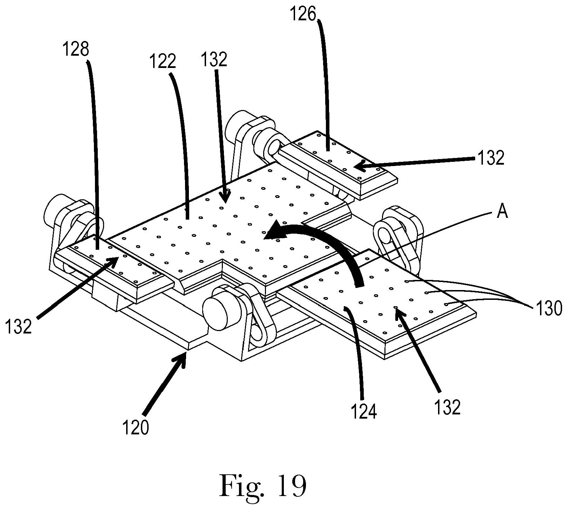

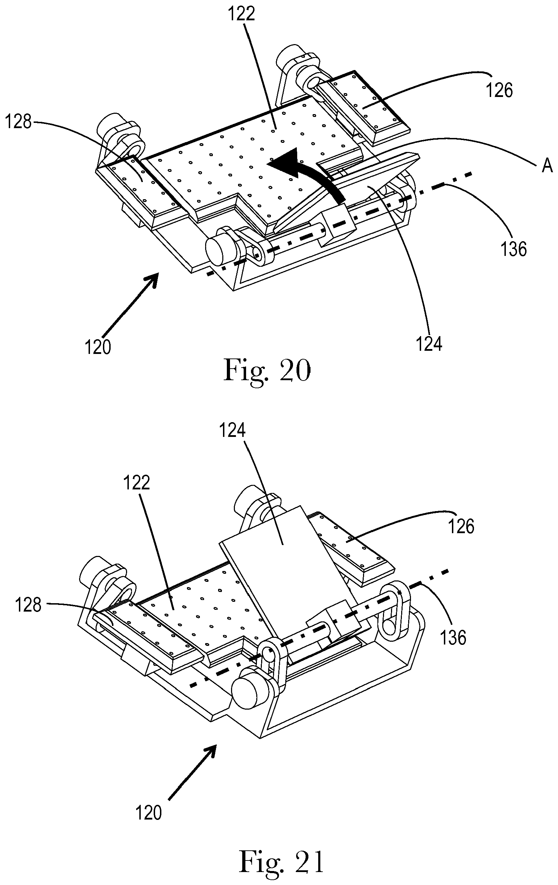

Referring to Figs. 8 and 10, the folding drum 112 continues to rotate the absorbent article to stage C where the folding device 120 may bi-fold the second waist region 20 of the chassis 12 into a face-to-face relationship with the first waist region 18 of the chassis 12. As shown in Figs. 19-22, to bi-fold the chassis 12, the chassis folding member 124 may pivot in a direction, A, radially outward from the outer surface 114 of the folding drum 112 and toward the chassis support member 122. The chassis folding member 124 may pivot about an axis of rotation 136 that is generally coincident with the lateral axis 26 of the chassis 12. The chassis folding member 124 pivots the second waist region 20 of the chassis 12 approximately 180 degrees into contact with the first waist region 18 of the chassis 12. As the chassis folding member 124 pivots in the direction, A, vacuum may be applied to the second waist region 20 of the chassis 12 through the zone 132 of vacuum apertures 130 in the chassis folding member 124 as shown in Fig. 19 to hold the second waist region 20 of the chassis 12 on the chassis folding member 124 during the bi-folding process. In addition, vacuum may be applied to the first waist region 18 of the chassis 112 through the zone 132 of vacuum apertures 130 in the chassis support member 122.

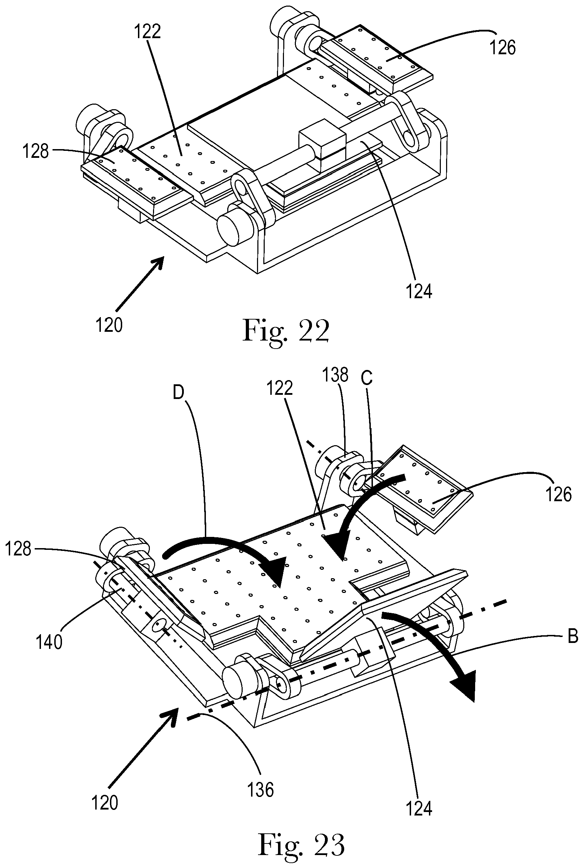

Once the second waist region 20 of the chassis 12 is folded into a face-to-face relationship with the first waist region 18 of the chassis 12, vacuum may be interrupted to the zone 132 of vacuum apertures 130 in the chassis folding member 124. In some exemplary configurations, compressed air may be directed through the vacuum apertures 130 and at the second waist region 20 of the chassis 12 to assist the second waist region 20 of the chassis 12 in separating from the chassis folding member 124. As shown in Fig. 23, once the second waist region 20 of the chassis 12 is in a face-to-face relationship with the first waist region 18, the chassis folding member 124 may pivot in a direction, B, about the axis of rotation 136, away from the chassis support member 122.

Referring to Figs. 8 and 11, from stage C, the folding drum 112 may rotate the absorbent article to stage D. In stage D, the folding device 120 folds the first and second side panels 28 and 30 of the absorbent article toward the second waist region 20 of the chassis 12. With reference to Figs. 4 and 23, to fold the first and second side panels 28 and 30, the first and second side panel folding members 126 and 128 may pivot in directions, C and D, respectively, radially outward from the outer surface 114 of the folding drum 112 and toward the chassis support member 122. The first and second side panel folding members 126 and 128 may pivot about axes of rotation 138 and 140, respectively, which are generally coincident with fold lines 32 of the first and second side panels 28 and 30 as shown in Fig. 10. Referring to Figs. 11 and 23, the first and second side panel folding members 126 and 128 pivot the second end region 35 of the first and second side panels 28 and 30 approximately 180 degrees into contact with the second waist region 20 of the chassis 12. As the first and second side panel folding members 126 and 128 pivot in the directions, C and D, vacuum may be applied to the first and second side panels 28 and 30 through the zones 132 of vacuum apertures 130 in the first and second side panel folding members 126 and 128 to hold the first and second side panels 28 and 30 on the first and second side panel folding members 126 and 128 during the side panel folding process.

It is to be appreciated that in some exemplary configurations, the second side panel folding member 128 may fold the second side panel 30 concurrently as the first side panel folding member 126 folds the first side panel 28. In other exemplary configurations, the second side panel folding member 128 may fold the second side panel 30 subsequent to the first side panel folding member 126 folding the first side panel 28.

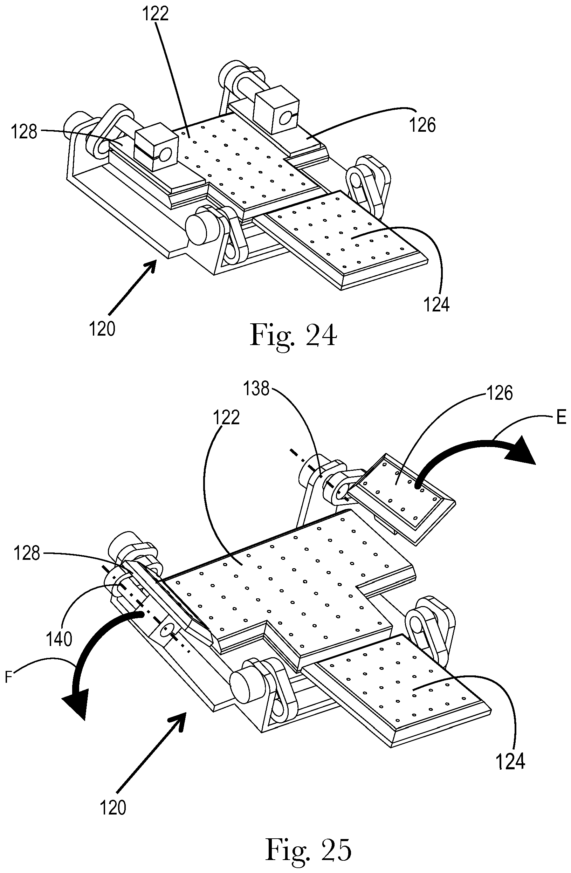

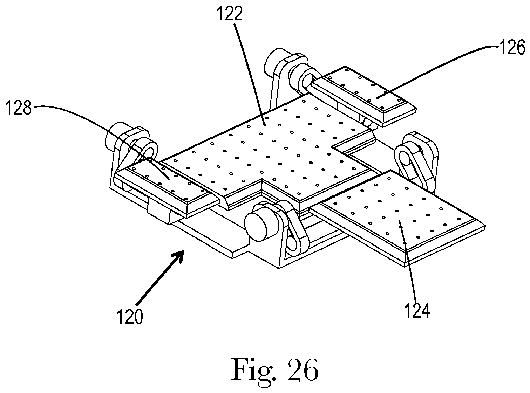

Referring to Figs. 11 and 24, once first and second side panel folding members 126 and 128 fold the second end region 35 of the first and second side panels 28 and 30 into a face-to-face relationship with the second waist region 20 of the chassis 12, vacuum may be interrupted to the zones 132 of vacuum apertures 130 in the first and second side panel folding members 126 and 128. In some exemplary configurations, compressed air may be directed through the vacuum apertures 130 and toward the second end regions 35 of the first and second side panels 28 and 30 to assist the first and second side panels 28 and 30 in separating from the first and second side panel folding members 126 and 128. Referring to Figs. 25 and 26, then, the first and second side panel folding members 126 and 128 may pivot in directions, E and F, about the axes of rotation 138 and 140, respectively, away from the chassis support member 122. In some exemplary configurations, stage D may occur prior to stage C. In other exemplary configurations, stages C and D may overlap.

After the first and second side panels 28 and 30 are folded, the absorbent article may rotate to stage E where the first and second side panels 28 and 30 are connected with the second waist region 20 of the chassis 12. The first and second side panels 28 and 30 may be refastenably connected with the second waist region 20 of the chassis 12. Refastenable methods of attachment include hook and loop fasteners and tape fasteners, for example. In other exemplary configurations, the first and second side panels 28 and 30 may be permanently connected with the second waist region 20 of the chassis 12. Permanent methods of attachment include heat bonding, pressure bonding, and the use of adhesives or cohesives, for example. Heat bonding may include hot air seaming, such as described in U.S. Patent No. 8,778,127, issued on July 15th 2014. It is to be appreciated that stage E is optional, as in some exemplary configurations, the first and second panels 28 and 30 may be connected with the second waist region 20 of the chassis 12 after the diaper pant 10 has been discharged from the folding drum 112.

With reference to Fig. 8, the diaper pant 10 may rotate to stage F where the diaper pant 10 is discharged from the folding drum 112. The diaper pant 10 may be discharged, for example, onto a third carrier apparatus 106. As shown in Fig. 8, the third carrier apparatus 106 may be in the form of a conveyor.

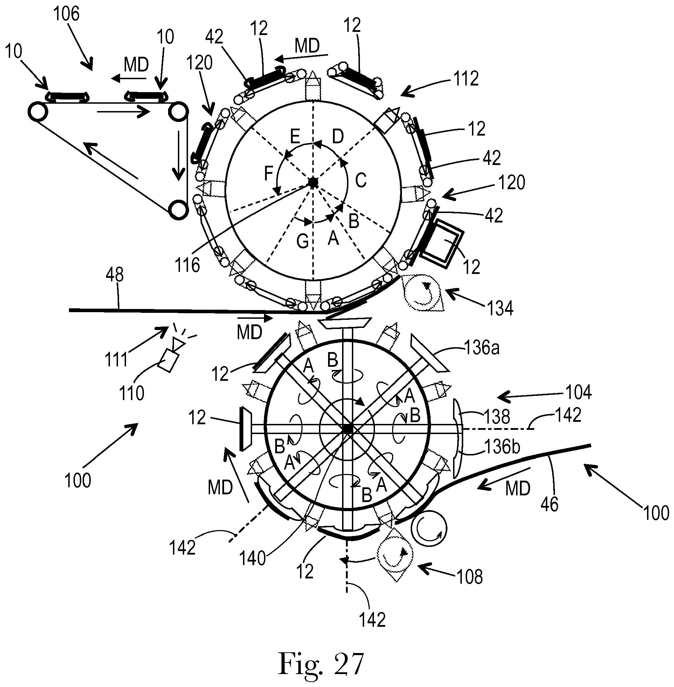

As discussed above with regard to Fig. 8, in some exemplary configurations, the chassis 12 and the continuous waist belt web 48 may be combined prior to advancing the continuous waist belt web 48 onto the folding drum 112. As shown in Fig. 27, in other exemplary configurations, the continuous waist belt web 48 and chassis 12 may be combined subsequent to the continuous waist belt web 48 advancing onto the folding drum 112. In such an exemplary configuration, the continuous waist belt web 48 may advance onto the outer surface 114 of the folding drum 112 in stage G. Next, a chassis 12 may advance in the machine direction MD on the second carrier apparatus 104 and may be combined with the continuous waist belt web 48 advancing on the folding drum 112 in stage A of Fig. 27.

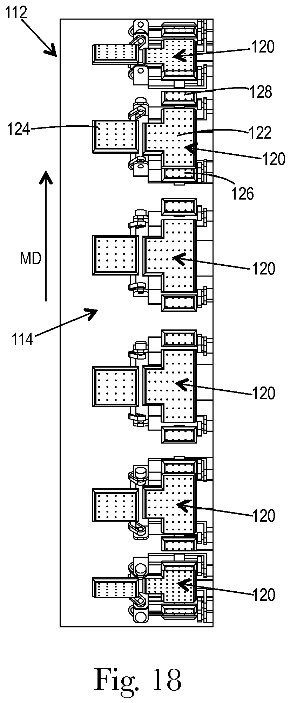

As discussed above, in some exemplary configurations, the folding drum 112 may comprise more than one lane of folding devices 118. For example, as shown in Figs. 16, 17, and 28, the folding drum 112 may comprise first and second lanes of folding devices 118a and 118b. As such, the folding drum 112 may be configured to concurrently fold multiple absorbent articles, thereby increasing the numbers of absorbent articles that the folding drum 112 may fold over a given time period compared to a folding apparatus 112 having only one lane 118 of folding devices 120 as shown in Fig. 18. Each folding device 120 in the first and second lanes 118a and 118b may include a chassis support member 122, a chassis folding member 124, a first side panel folding member 126, and a second side panel folding member 128 as shown in Figs. 16, 17, and 28. The folding devices 120 of the first lane 118a may be staggered from the folding devices 120 of the second lane 118b. For example, a chassis folding member 124 of the first lane 118a of folding devices 120 may be positioned between two chassis folding members 124 of the second lane 118b of folding devices 120. Likewise, a chassis folding member 124 of the second lane 118b of folding devices 120 may be positioned between two chassis folding members 124 of the first lane 118a of folding devices 120. That is, the folding devices 120 in the first lane 118a may be offset from the folding devices 120 in the second lane 118b in a direction parallel with the axis of rotation 116 of the folding drum 112.

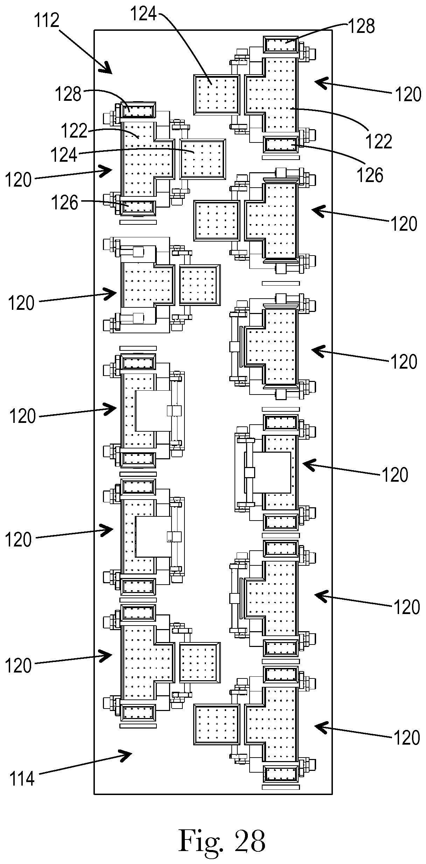

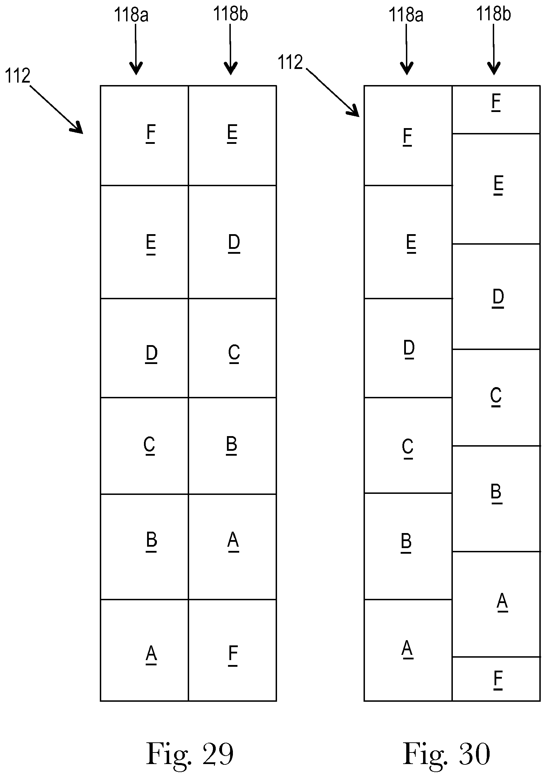

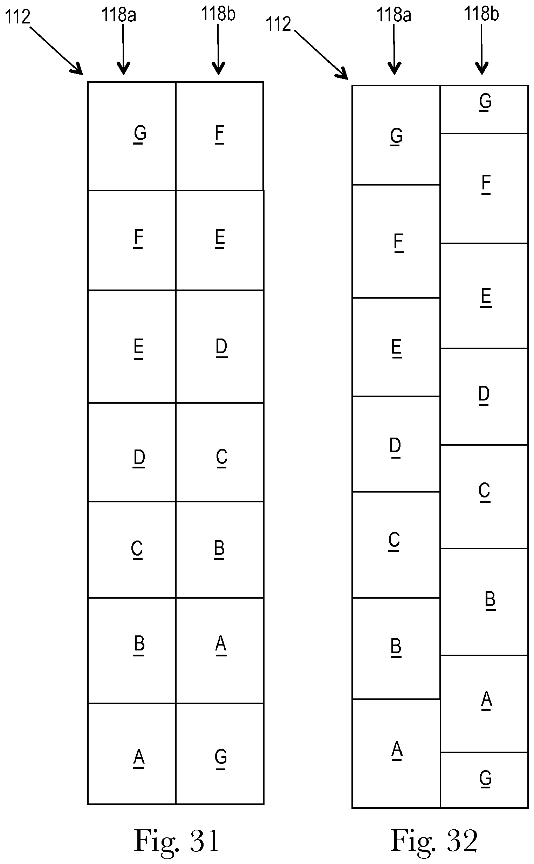

As previously mentioned, a folding drum 112 comprising first and second lanes 118a and 118b of folding devices 120 such as shown in Figs. 16, 17, and 28, may operate similar to a folding drum 112 comprising a single lane 118 of folding devices 120 such as shown in Fig. 18. For example, with reference to Figs. 28-30, the folding drum 112 may rotate the absorbent articles in the first and second lanes 118a and 118b through stages A-F discussed above. The stages A-F that the absorbent article rotates through in the second lane 118b may be sequential to the stages A-F that the absorbent article rotates through in the first lane 118a such as shown in Fig. 29. For example, a folding device 120 in the first lane 118a may rotate through stage B at the same time as an adjacent folding device 120 in the second lane 118b, which is located upstream of the folding device 120 in the first lane, rotates through stage A. In other exemplary configurations, the stages A-F that the absorbent article rotates through in second lane 118b may partially overlap the stages A-F that the absorbent article rotates through in the first lane 118a such as shown in Fig. 30. For example, a folding device 120 in the first lane 118a may rotate through stage A while an adjacent folding device 120 in the second lane 118a, which is located upstream of the folding device 120 in the first lane 118a, begins rotating through stage A. In such an exemplary configuration, the folding device 120 in the first lane 118a may rotate onto stage B as the folding device 120 in the second lane 118b continues rotating through stage B. As shown in Figs. 31 and 32, in an exemplary configuration having first and second lanes 118a and 118b of folding devices, the folding drum 112 may rotate absorbent article in each lane through stages A-G as discussed above with regard to Fig. 27. Stages A-G may occur sequentially as shown in Fig. 31, or may overlap as shown in Fig. 32. As discussed above with regard to Fig. 8, the stages A-G may be performed in different order. In some exemplary configurations, stages A-G may partially overlap each other. In some exemplary configurations, a folding drum 112 may not rotate an absorbent article through all of the stages A-G.

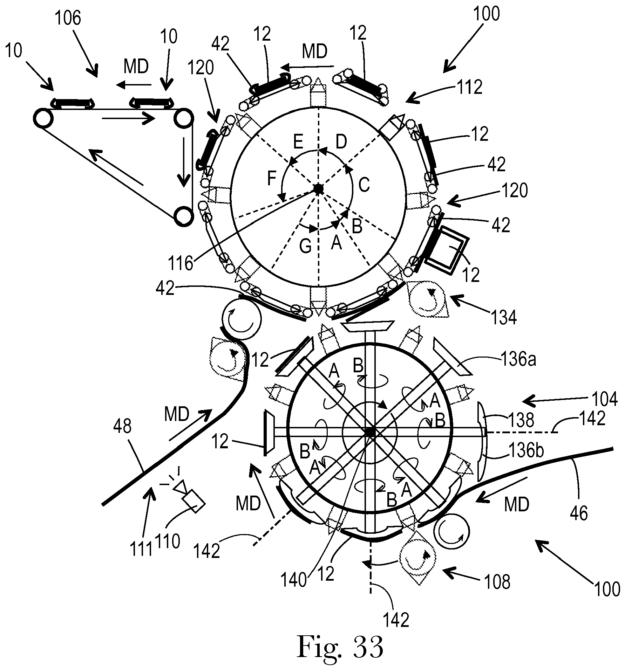

As shown in Fig 33, in some exemplary configurations, the continuous waist belt web 48 may be cut into discrete waist belts 42 prior to advancing on the folding drum 112. In some exemplary configurations, the discrete waist belts 42 may advance onto the folding drum 112 and may be combined with chassis 12. It is to be appreciated that the discrete waist belts 42 may advance onto the folding drum 112 prior to, or subsequent to, the chassis 12 advancing on to the folding drum 112.

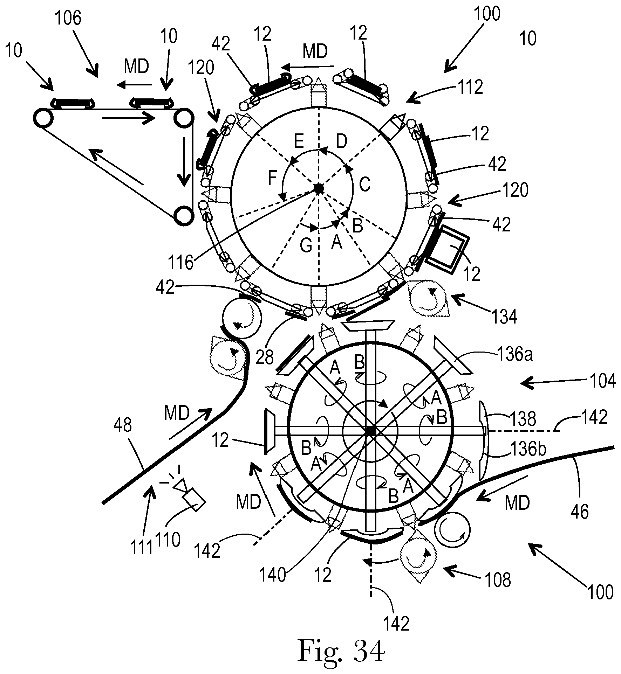

As shown in Fig. 34, in other exemplary configurations, the continuous waist belt web 48 may be cut into first and second side panels 28 and 30 such as described above with regard to Fig. 5. The first and second side panels 28 and 30 may advance to the folding drum 112 and may be combined with chassis 12. It is to be appreciated that the first and second side panels 28 and 30 may advance onto the folding drum 112 prior to, or subsequent to, the chassis 12 advancing on to the folding drum 112.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A method for assembling disposable diaper pants (10), each diaper pant comprising a chassis (12), a first side panel (28), and a second side panel (30), each chassis (12) defining a longitudinal axis (24) and a lateral axis (26), the chassis (12) having a first waist region (18), a second waist region (20), and a crotch region (22) positioned longitudinally between the first and second waist regions (18, 20), the method comprising:
rotating a drum (112) about an axis of rotation (116), the drum (112) comprising a first lane of folding devices (118a) on an outer surface (114) of the drum (112) and a second lane of folding devices (118b) on the outer surface (114) of the drum (112), wherein each folding device comprises: a chassis support member (122), a chassis folding member (124), a first side panel folding member (126), and a second side panel folding member (128);
advancing first and second side panels (28, 30) in a machine direction to the first lane of folding devices (118a), wherein the first side panel (28) is positioned on the first side panel folding member (126) and the second side panel (30) is positioned on the second side panel folding member (128);
advancing a first chassis (12a) in the machine direction to the first lane of folding devices (118a), wherein the first waist region (18) of the first chassis (12a) is positioned on the chassis support member (122) and the second waist region (20) of the first chassis (12a) is positioned on the chassis folding member (124);
connecting first end regions of the first and second side panels (28, 30) with the first waist region of the first chassis (12a);
advancing third and fourth side panels in the machine direction to the second lane of folding devices (118b), wherein the third side panel is positioned on the first side panel folding member (126) and the fourth side panel is positioned on the second side panel folding member (128);
advancing a second chassis (12b) in the machine direction to the second lane of folding devices (118b), wherein the first waist region of the second chassis is positioned on the chassis support member (122) and the second waist region of the second chassis (12b) is positioned on the chassis folding member (124);
connecting first end regions of the third and fourth side panels with the first waist region of the second chassis (12b);
pivoting the chassis folding member (124) of the first lane of folding devices (118a) relative to the chassis support member (122) to position the first and second waist regions of the first chassis (12a) into a face-to-face relationship;
pivoting the chassis folding member (124) of the second lane of folding devices (118b) relative to the chassis support member (122) to position the first and second waist regions of the second chassis (12b) into a face-to-face relationship;
pivoting the first and second side panel folding members (126, 128) of the first lane of folding devices (118a) relative to the chassis support member (122) to position the first and second side panels (28, 30) into contact with the second waist region of the first chassis (12a);
pivoting the first and second side panel folding members (126, 128) of the second lane of folding devices (118b) relative to the chassis support member (122) to position the third and fourth side panels into contact with the second waist region of the second chassis;
connecting the first and second side panels (28, 30) with the second waist region of the first chassis (12a) to form a first diaper pant; and
connecting the second and third side panels with the second waist region of the second chassis (12a) to form a second diaper pant.

2. The method according to Claim 1 further comprising the steps of:
advancing a first continuous web in the machine direction to the first lane of folding devices (118a) on the drum (112), wherein a portion of the first continuous web positioned on the first side panel folding member (126) defines the first side panel (28) and a portion of the first continuous web positioned on the second side panel folding member (128) defines the second side panel (30);
advancing a second continuous web in the machine direction to the second lane of folding devices (118b) on the drum (112), wherein a portion of the second continuous web positioned on the first side panel folding member (126) defines the third side panel and a portion of the second continuous web positioned on the second side panel folding member (128) defines the fourth side panel; and
cutting the first and second continuous webs into discrete waist belts (42).

3. The method according to any of the preceding claims, wherein the step of connecting first end regions of the first and second side panels (28, 30) with the first waist region (18) of the chassis (12) occurs prior to the step of advancing first and second side panels (28, 30) in a machine direction to the first lane of folding devices (118a).

4. The method according to any of the preceding claims, wherein the first and second side panels (28, 30) are permanently connected with the second waist region (20) of the chassis (12).

5. The method according to any of Claims 1 to 3, wherein the first and second side panels (28, 30) are refastenably connected with the second waist region (20) of the chassis (12).

6. The method according to any of the preceding claims further comprising the step of applying vacuum pressure to hold the first and second chassis (12a, 12b) on the chassis support members (122) and the chassis folding members (124).

7. The method according to any of the preceding claims further comprising the steps of:
orienting the first chassis (12a) such that the lateral axis (26) is parallel with the machine direction prior to the step of connecting first end regions of the first and second side panels (28, 30) with the first waist region (18) of the first chassis (12a); and
orienting the second chassis (12b) such that the lateral axis (26) is parallel with the machine direction prior to the step of connecting first end regions of the third and fourth side panels with the first waist region (18) of the second chassis (12b).

8. An apparatus for assembling diaper pants (10) comprising a drum (112) rotatable about an axis of rotation (116), the drum (112) having an outer surface (114), the outer surface (114) comprising
a first lane of folding devices (118a), each folding device comprising:
a chassis support member (122);
a chassis folding member (124) pivotally connected with the chassis support member (122);
a first side panel folding member (126) pivotally connected with the chassis support member (122); and
a second side panel folding member (128) pivotally connected with the chassis support member (122);
a second lane of folding devices (118b), each folding device comprising:
a chassis support member (122);
a chassis folding member (124) pivotally connected with the chassis support member (122);
a first side panel folding member (126) pivotally connected with the chassis support member (122); and
a second side panel folding member (128) pivotally connected with the chassis support member (122),
wherein the first lane of folding devices (118a) is offset from the second lane of folding devices (118b) in a direction parallel to the axis of rotation (116),
wherein the chassis folding member (124) of the first lane of folding device (118a) is positioned between two chassis folding members (124) of the second lane of folding devices (118b),
wherein the chassis support members (122), chassis folding members (124), first side panel folding members (126), and second side panel folding members (128) of the first and second lane of folding devices (118a, 118b) each comprise a plurality of vacuum apertures (130), and further comprising a carrier apparatus positioned adjacent to the drum (112), wherein the carrier apparatus rotates about a first axis of rotation (140), wherein the carrier apparatus comprises a plurality of first carrier members separated by a plurality of second carrier apparatus, wherein each carrier member comprises an outer surface, wherein the first carrier members (102) rotate in a first direction about a second axis of rotation (142), wherein the second carrier members (104) rotate in a second direction about the second axis of rotation (142), wherein the first direction is opposite the second direction, and wherein the first axis of rotation (140) is orthogonal to the second axis of rotation (142).

## Patentansprüche

1. Verfahren zum Zusammenfügen von Einweg-Windelhöschen (10), wobei jedes Windelhöschen eine Grundeinheit (12), ein erstes Seitenfeld (28) und ein zweites Seitenfeld (30) umfasst, wobei jede Grundeinheit (12) eine Längsachse (24) und eine Querachse (26) bestimmt, wobei die Grundeinheit (12) einen ersten Taillenbereich (18), einen zweiten Taillenbereich (20) und einen Schrittbereich (22), der längs zwischen dem ersten und dem zweiten Taillenbereich (18, 20) angeordnet ist, aufweist, wobei das Verfahren umfasst:
Drehen einer Walze (112) um eine Drehachse (116), wobei die Walze (112) eine erste Bahn von Faltvorrichtungen (118a) auf einer Außenoberfläche (114) der Walze (112) und eine zweite Bahn von Faltvorrichtungen (118b) auf der Außenoberfläche (114) der Walze (112) umfasst, wobei jede Faltvorrichtung umfasst: ein Grundeinheit-Auflageelement (122), ein Grundeinheit-Faltelement (124), ein erstes Seitenfeld-Faltelement (126) und ein zweites Seitenfeld-Faltelement (128);
Vorwärtsbewegen des ersten und des zweiten Seitenfelds (28, 30) in einer Maschinenlaufrichtung zu der ersten Bahn von Faltvorrichtungen (118a), wobei das erste Seitenfeld (28) auf dem ersten Seitenfeld-Faltelement (126) angeordnet wird und das zweite Seitenfeld (30) auf dem zweiten Seitenfeld-Faltelement (128) angeordnet wird;
Vorwärtsbewegen einer ersten Grundeinheit (12a) in der Maschinenlaufrichtung zu der ersten Bahn von Faltvorrichtungen (118a), wobei der erste Taillenbereich (18) der ersten Grundeinheit (12a) auf dem Grundeinheit-Auflageelement (122) angeordnet wird und der zweite Taillenbereich (20) der ersten Grundeinheit (12a) auf dem Grundeinheit-Faltelement (124) angeordnet wird;
Verbinden von ersten Endbereichen des ersten und des zweiten Seitenfelds (28, 30) mit dem ersten Taillenbereich der ersten Grundeinheit (12a);
Vorwärtsbewegen eines dritten und eines vierten Seitenfelds in der Maschinenlaufrichtung zu der zweiten Bahn von Faltvorrichtungen (118b), wobei das dritte Seitenfeld auf dem ersten Seitenfeld-Faltelement (126) angeordnet wird und das vierte Seitenfeld auf dem zweiten Seitenfeld-Faltelement (128) angeordnet wird;
Vorwärtsbewegen einer zweiten Grundeinheit (12b) in der Maschinenlaufrichtung zu der zweiten Bahn von Faltvorrichtungen (118b), wobei der erste Taillenbereich der zweiten Grundeinheit auf dem Grundeinheit-Auflageelement (122) angeordnet wird und der zweite Taillenbereich der zweiten Grundeinheit (12b) auf dem Grundeinheit-Faltelement (124) angeordnet wird;
Verbinden von ersten Endbereichen des dritten und des vierten Seitenfelds mit dem ersten Taillenbereich der zweiten Grundeinheit (12b);
Drehen des Grundeinheit-Faltelements (124) der ersten Bahn von Faltvorrichtungen (118a) relativ zu dem Grundeinheit-Auflageelement (122), um den ersten und den zweiten Taillenbereich der ersten Grundeinheit (12a) in einer gegenüberliegenden Beziehung anzuordnen;
Drehen des Grundeinheit-Faltelements (124) der zweiten Bahn von Faltvorrichtungen (118b) relativ zu dem Grundeinheit-Auflageelement (122), um den ersten und den zweiten Taillenbereich der zweiten Grundeinheit (12b) in einer gegenüberliegenden Beziehung anzuordnen;
Drehen des ersten und des zweiten Seitenfeld-Faltelements (126, 128) der ersten Bahn von Faltvorrichtungen (118a) relativ zu dem Grundeinheit-Auflageelement (122), um das erste und das zweite Seitenfeld (28, 30) in Kontakt mit dem zweiten Taillenbereich der ersten Grundeinheit (12a) anzuordnen;
Drehen des ersten und des zweiten Seitenfeld-Faltelements (126, 128) der zweiten Bahn von Faltvorrichtungen (118b) relativ zu dem Grundeinheit-Auflageelement (122), um das dritte und das vierte Seitenfeld in Kontakt mit dem zweiten Taillenbereich der zweiten Grundeinheit anzuordnen;
Verbinden des ersten und des zweiten Seitenfelds (28, 30) mit dem zweiten Taillenbereich der ersten Grundeinheit (12a), um ein erstes Windelhöschen zu bilden; und
Verbinden des zweiten und des dritten Seitenfelds mit dem zweiten Taillenbereich der zweiten Grundeinheit (12b), um ein zweites Windelhöschen zu bilden.

2. Verfahren nach Anspruch 1, ferner die folgenden Schritte umfassend:
Vorwärtsbewegen einer ersten Endlosbahn in der Maschinenlaufrichtung zu der ersten Bahn von Faltvorrichtungen (118a) auf der Walze (112), wobei ein auf dem ersten Seitenfeld-Faltelement (126) angeordneter Abschnitt der ersten Endlosbahn das erste Seitenfeld (28) bestimmt und ein auf dem zweiten Seitenfeld-Faltelement (128) angeordneter Abschnitt der ersten Endlosbahn das zweite Seitenfeld (30) bestimmt;
Vorwärtsbewegen einer zweiten Endlosbahn in der Maschinenlaufrichtung zu der zweiten Bahn von Faltvorrichtungen (118b) auf der Walze (112), wobei ein auf dem ersten Seitenfeld-Faltelement (126) angeordneter Abschnitt der zweiten Endlosbahn das dritte Seitenfeld bestimmt und ein auf dem zweiten Seitenfeld-Faltelement (128) angeordneter Abschnitt der zweiten Endlosbahn das vierte Seitenfeld bestimmt; und
Schneiden der ersten und der zweiten Endlosbahn in separate Taillenbänder (42).

3. Verfahren nach einem der vorstehenden Ansprüche, wobei der Schritt des Verbindens von ersten Endbereichen des ersten und des zweiten Seitenfelds (28, 30) mit dem ersten Taillenbereich (18) der Grundeinheit (12) vor dem Schritt des Vorwärtsbewegens des ersten und des zweiten Seitenfelds (28, 30) in einer Maschinenlaufrichtung zu der ersten Bahn von Faltvorrichtungen (118a) erfolgt.

4. Verfahren nach einem der vorstehenden Ansprüche, wobei das erste und das zweite Seitenfeld (28, 30) dauerhaft mit dem zweiten Taillenbereich (20) der Grundeinheit (12) verbunden werden.

5. Verfahren nach einem der Ansprüche 1 bis 3, wobei das erste und das zweite Seitenfeld (28, 30) wiederverschließbar mit dem zweiten Taillenbereich (20) der Grundeinheit (12) verbunden werden.

6. Verfahren nach einem der vorstehenden Ansprüche, ferner umfassend den Schritt des Anwendens von Vakuumdruck, um die erste und die zweite Grundeinheit (12a, 12b) auf den Grundeinheit-Auflageelementen (122) und den Grundeinheit-Faltelementen (124) zu halten.

7. Verfahren nach einem der vorstehenden Ansprüche, ferner die folgenden Schritte umfassend:
Ausrichten der ersten Grundeinheit (12a) derart, dass die Querachse (26) vor dem Schritt des Verbindens von ersten Endbereichen des ersten und des zweiten Seitenfelds (28, 30) mit dem ersten Taillenbereich (18) der ersten Grundeinheit (12a) parallel zur Maschinenlaufrichtung verläuft; und
Ausrichten der zweiten Grundeinheit (12b) derart, dass die Querachse (26) vor dem Schritt des Verbindens von ersten Endbereichen des dritten und des vierten Seitenfelds mit dem ersten Taillenbereich (18) der zweiten Grundeinheit (12b) parallel zur Maschinenlaufrichtung verläuft.

8. Vorrichtung zum Zusammenfügen von Windelhöschen (10), umfassend eine Walze (112), die um eine Drehachse (116) drehbar ist, wobei die Walze (112) eine Außenoberfläche (114) aufweist, wobei die Außenoberfläche (114) umfasst:
eine erste Bahn von Faltvorrichtungen (118a), wobei jede Faltvorrichtung umfasst:
ein Grundeinheit-Auflageelement (122);
ein Grundeinheit-Faltelement (124), das drehbar mit dem Grundeinheit-Auflageelement (122) verbunden ist;
ein erstes Seitenfeld-Faltelement (126), das drehbar mit dem Grundeinheit-Auflageelement (122) verbunden ist; und
ein zweites Seitenfeld-Faltelement (128), das drehbar mit dem Grundeinheit-Auflageelement (122) verbunden ist;
eine zweite Bahn von Faltvorrichtungen (118b), wobei jede Faltvorrichtung umfasst:
ein Grundeinheit-Auflageelement (122);
ein Grundeinheit-Faltelement (124), das drehbar mit dem Grundeinheit-Auflageelement (122) verbunden ist;
ein erstes Seitenfeld-Faltelement (126), das drehbar mit dem Grundeinheit-Auflageelement (122) verbunden ist; und
ein zweites Seitenfeld-Faltelement (128), das drehbar mit dem Grundeinheit-Auflageelement (122) verbunden ist,
wobei die erste Bahn von Faltvorrichtungen (118a) von der zweiten Bahn von Faltvorrichtungen (118b) in einer Richtung parallel zur Drehachse (116) versetzt ist,
wobei das Grundeinheit-Faltelement (124) der ersten Bahn von Faltvorrichtungen (118a) zwischen zwei Grundeinheit-Faltelementen (124) der zweiten Bahn von Faltvorrichtungen (118b) angeordnet ist,
wobei die Grundeinheit-Auflageelemente (122), Grundeinheit-Faltelemente (124), ersten Seitenfeld-Faltelemente (126) und zweiten Seitenfeld-Faltelemente (128) der ersten und der zweiten Bahn von Faltvorrichtungen (118a, 118b) jeweils eine Vielzahl von Vakuumöffnungen (130) umfassen, und
ferner umfassend eine Trägervorrichtung, die benachbart zu der Walze (112) angeordnet ist, wobei sich die Trägervorrichtung um eine erste Drehachse (140) dreht, wobei die Trägervorrichtung mehrere erste Trägerelemente umfasst, die durch mehrere zweite Trägervorrichtungen getrennt sind, wobei jedes Trägerelement eine Außenoberfläche umfasst, wobei sich die ersten Trägerelemente (102) in einer ersten Richtung um eine zweite Drehachse (142) drehen, wobei sich die zweiten Trägerelemente (104) in einer zweiten Richtung um die zweite Drehachse (142) drehen, wobei die erste Richtung der zweiten Richtung entgegengesetzt ist, und wobei die erste Drehachse (140) senkrecht zu der zweiten Drehachse (142) ist.

## Revendications

1. Procédé d'assemblage de couches-culottes jetables (10), chaque couche-culotte comprenant un châssis (12), un premier pan latéral (28) et un deuxième pan latéral (30), chaque châssis (12) définissant un axe longitudinal (24) et un axe latéral (26), le châssis (12) comportant une première région de ceinture (18), une deuxième région de ceinture (20) et une région d'entrejambe (22) positionnée longitudinalement entre les première et deuxième régions de ceinture (18, 20), le procédé comprenant :
la rotation d'un tambour (112) autour d'un axe de rotation (116), le tambour (112) comprenant une première voie de dispositifs de pliage (118a) sur une surface externe (114) du tambour (112) et une deuxième voie de dispositifs de pliage (118b) sur la surface externe (114) du tambour (112), dans lequel chaque dispositif de pliage comprend : un élément de support de châssis (122), un élément de pliage de châssis (124), un premier élément de pliage de pan latéral (126) et un deuxième élément de pliage de pan lateral (128) ;
l'avancement de premier et deuxième pans latéraux (28, 30) dans une direction de la machine vers la première voie de dispositifs de pliage (118a), dans lequel le premier pan latéral (28) est positionné sur le premier élément de pliage de pan latéral (126) et le deuxième pan latéral (30) est positionné sur le deuxième élément de pliage de pan latéral (128) ;
l'avancement d'un premier châssis (12a) dans la direction de la machine vers la première voie de dispositifs de pliage (118a), dans lequel la première région de ceinture (18) du premier châssis (12a) est positionnée sur l'élément de support de châssis (122) et la deuxième région de ceinture (20) du premier châssis (12a) est positionnée sur l'élément de pliage de châssis (124) ;
le raccordement des premières régions d'extrémité des premier et deuxième pans latéraux (28, 30) à la première région de ceinture du premier châssis (12a) ;
l'avancement de troisième et quatrième pans latéraux dans la direction de la machine vers la deuxième voie de dispositifs de pliage (118b), dans lequel le troisième pan latéral est positionné sur le premier élément de pliage de pan latéral (126) et le quatrième pan latéral est positionné sur le deuxième élément de pliage de pan latéral (128) ;
l'avancement d'un deuxième châssis (12b) dans la direction de la machine vers la deuxième voie de dispositifs de pliage (118b), dans lequel la première région de ceinture du deuxième châssis est positionnée sur l'élément de support de châssis (122) et la deuxième région de ceinture du deuxième châssis (12b) est positionnée sur l'élément de pliage de châssis (124) ;
le raccordement des premières régions d'extrémité des troisième et quatrième pans latéraux à la première région de ceinture du deuxième châssis (12b) ;
le pivotement de l'élément de pliage de châssis (124) de la première voie de dispositifs de pliage (118a) par rapport à l'élément de support de châssis (122) pour positionner les première et deuxième régions de ceinture du premier châssis (12a) dans une relation face à face ;
le pivotement de l'élément de pliage de châssis (124) de la deuxième voie de dispositifs de pliage (118b) par rapport à l'élément de support de châssis (122) pour positionner les première et deuxième régions de ceinture du deuxième châssis (12b) dans une relation face à face ;
le pivotement des premier et deuxième éléments de pliage de pan latéral (126, 128) de la première voie de dispositifs de pliage (118a) par rapport à l'élément de support de châssis (122) pour positionner les premier et deuxième pans latéraux (28, 30) en contact avec la deuxième région de ceinture du premier châssis (12a) ;
le pivotement des premier et deuxième éléments de pliage de pan latéral (126, 128) de la deuxième voie de dispositifs de pliage (118b) par rapport à l'élément de support de châssis (122) pour positionner les troisième et quatrième pans latéraux en contact avec la deuxième région de ceinture du deuxième châssis ;
le raccordement des premier et deuxième pans latéraux (28, 30) à la deuxième région de ceinture du premier châssis (12a) pour former une première couche-culotte ; et
le raccordement des deuxième et troisième pans latéraux à la deuxième région de ceinture du deuxième châssis (12a) pour former une deuxième couche-culotte.

2. Procédé selon la revendication 1, comprenant en outre les étapes consistant à:
faire avancer une première nappe continue dans la direction de la machine vers la première voie de dispositifs de pliage (118a) sur le tambour (112), dans lequel une partie de la première nappe continue positionnée sur le premier élément de pliage de pan latéral (126) définit le premier pan latéral (28) et une partie de la première nappe continue positionnée sur le deuxième élément de pliage de pan latéral (128) définit le deuxième pan latéral (30) ;
faire avancer une deuxième nappe continue dans la direction de la machine vers la deuxième voie de dispositifs de pliage (118b) sur le tambour (112), dans lequel une partie de la deuxième nappe continue positionnée sur le premier élément de pliage de pan latéral (126) définit le troisième pan latéral et une partie de la deuxième nappe continue positionnée sur le deuxième élément de pliage de pan latéral (128) définit le quatrième pan latéral ; et
couper les première et deuxième nappes continues en ceintures de taille distinctes (42).

3. Procédé selon l'une quelconque des revendications précédentes, dans lequel l'étape de raccordement des premières régions d'extrémité des premier et deuxième pans latéraux (28, 30) à la première région de ceinture (18) du châssis (12) se produit avant l'étape consistant à faire avancer les premier et deuxième pans latéraux (28, 30) dans une direction de la machine vers la première voie de dispositifs de pliage (118a).

4. Procédé selon l'une quelconque des revendications précédentes, dans lequel les premier et deuxième pans latéraux (28, 30) sont raccordés de manière permanente à la deuxième région de ceinture (20) du châssis (12).

5. Procédé selon l'une quelconque des revendications 1 à 3, dans lequel les premier et deuxième pans latéraux (28, 30) sont raccordés de manière rattachable à la deuxième région de ceinture (20) du châssis (12).

6. Procédé selon l'une quelconque des revendications précédentes, comprenant en outre l'étape d'application d'une dépression pour maintenir les premier et deuxième châssis (12a, 12b) sur les éléments de support de châssis (122) et les éléments de pliage de châssis (124).

7. Procédé selon l'une quelconque des revendications précédentes, comprenant en outre les étapes consistant à :
orienter le premier châssis (12a) de telle sorte que l'axe latéral (26) est parallèle à la direction de la machine avant l'étape de raccordement des premières régions d'extrémité des premier et deuxième pans latéraux (28, 30) à la première région de ceinture (18) du premier châssis (12a) ; et
orienter le deuxième châssis (12b) de telle sorte que l'axe latéral (26) est parallèle à la direction de la machine avant l'étape de raccordement des premières régions d'extrémité des troisième et quatrième pans latéraux à la première région de ceinture (18) du deuxième châssis (12b).

8. Appareil pour assembler des couches-culottes (10) comprenant un tambour (112) pouvant tourner autour d'un axe de rotation (116), le tambour (112) comportant une surface externe (114), la surface externe (114) comprenant
une première voie de dispositifs de pliage (118a), chaque dispositif de pliage comprenant:
un élément de support de châssis (122) ;
un élément de pliage de châssis (124) raccordé de façon pivotante à l'élément de support de châssis (122) ;
un premier élément de pliage de pan latéral (126) raccordé de façon pivotante à l'élément de support de châssis (122) ; et
un deuxième élément de pliage de pan latéral (128) raccordé de façon pivotante à l'élément de support de châssis (122) ;
une deuxième voie de dispositifs de pliage (118b), chaque dispositif de pliage comprenant :
un élément de support de chassis (122) ;
un élément de pliage de châssis (124) raccordé de façon pivotante à l'élément de support de châssis (122) ;
un premier élément de pliage de pan latéral (126) raccordé de façon pivotante à l'élément de support de châssis (122) ; et
un deuxième élément de pliage de pan latéral (128) raccordé de façon pivotante à l'élément de support de châssis (122),
dans lequel la première voie de dispositifs de pliage (118a) est décalée de la deuxième voie de dispositifs de pliage (118b) dans une direction parallèle à l'axe de rotation (116),
dans lequel l'élément de pliage de châssis (124) de la première voie de dispositifs de pliage (118a) est positionné entre deux éléments de pliage de châssis (124) de la deuxième voie de dispositifs de pliage (118b),
dans lequel les éléments de support de châssis (122), les éléments de pliage de châssis (124), les premiers éléments de pliage de pan latéral (126) et les deuxièmes éléments de pliage de pan latéral (128) des première et deuxième voies de dispositifs de pliage (118a, 118b) comprennent chacun une pluralité d'ouvertures à vide (130), et
comprenant en outre un appareil de support positionné à proximité du tambour (112), dans lequel l'appareil de support tourne autour d'un premier axe de rotation (140), dans lequel l'appareil de support comprend une pluralité de premiers éléments de support séparés par une pluralité de deuxièmes appareils de support, dans lequel chaque élément de support comprend une surface externe, dans lequel les premiers éléments de support (102) tournent dans une première direction autour d'un deuxième axe de rotation (142), dans lequel les deuxièmes éléments de support (104) tournent dans une deuxième direction autour du deuxième axe de rotation (142), dans lequel la première direction est opposée à la deuxième direction, et dans lequel le premier axe de rotation (140) est orthogonal au deuxième axe de rotation (142).
